(19) Europäisches Patentamt
European Patent Office
Office européen des brevets

(11) **EP 2 128 205 B1**

(12) **EUROPÄISCHE PATENTSCHRIFT**

(45) Veröffentlichungstag und Bekanntmachung des Hinweises auf die Patenterteilung:
**03.08.2016   Patentblatt 2016/31**

(21) Anmeldenummer: **09003175.8**

(22) Anmeldetag: **05.03.2009**

(51) Int Cl.:
*C09C 1/62* (2006.01)     *C09C 1/66* (2006.01)
*C09D 5/36* (2006.01)     *C09D 7/12* (2006.01)
*C09D 11/02* (2006.01)     *A61K 8/11* (2006.01)
*A61Q 1/02* (2006.01)     *B82Y 5/00* (2011.01)
*A61K 8/19* (2006.01)     *C09D 11/037* (2014.01)

(54) **MISCHUNG VON KUPFERHALTIGEM METALLEFFEKTPIGMENT UND VERFAHREN ZU DEREN HERSTELLUNG**

MIXTURE OF METAL EFFECT PIGMENTS CONTAINING COPPER AND METHOD FOR THEIR PRODUCTION

MÉLANGE DE PIGMENTS MÉTALLIQUES CONTENANT DU CUIVRE ET PROCÉDÉ DE FABRICATION

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **28.05.2008   EP 08009699**

(43) Veröffentlichungstag der Anmeldung:
**02.12.2009   Patentblatt 2009/49**

(73) Patentinhaber: **Eckart GmbH**
**91235 Hartenstein (DE)**

(72) Erfinder:
• **Becker, Michael, Dr.**
**91207 Lauf (DE)**
• **Wczasek, Katrin**
**90419 Nürnberg (DE)**

• **Heitmar, Svea**
**91284 Neuhaus/Pegnitz (DE)**
• **Prölß, Dieter**
**91126 Schwabach (DE)**

(74) Vertreter: **Louis Pöhlau Lohrentz**
**Patentanwälte**
**Postfach 30 55**
**90014 Nürnberg (DE)**

(56) Entgegenhaltungen:
**EP-A- 1 553 144     WO-A-2004/026972**
**WO-A-2006/066825     WO-A-2008/077612**
**DE-A1- 10 315 775     GB-A- 994 409**
**US-A- 2 864 719     US-A- 4 884 754**
**US-A1- 2002 050 186     US-A1- 2004 194 663**
**US-B1- 6 398 861**

**Beschreibung**

Die vorliegende Erfindung betrifft eine Mischung von kupferhaltigen Metalleffektpigmenten mit wenigstens einer weiteren Komponente, ein Beschichtungsmittel, das diese Mischung enthält, sowie einen diese Mischung oder dieses Beschichtungsmittel aufweisenden Gegenstand. Ferner betrifft die Erfindung ein Verfahren zur Herstellung einer Mischung von kupferhaltigen Metalleffektpigmenten mit wenigstens einer weiteren Komponente.

Kupferhaltige Metalleffektpigmente, umfassend Kupferpigmente oder aus einer Kupfer-Zink-Legierung hergestellte Messingpigmente, auch als Goldbronzepigmente bezeichnet, werden u. a. auch in der grafischen Industrie, beispielsweise in Druckfarben, eingesetzt.

Die bisher zur Pigmentierung von Flexo- und Tiefdruckfarben eingesetzten, durch Vermahlung aus Kupfer- oder Messinggrieß hergestellten Metalleffektpigmente, wie beispielsweise Goldbronzepigment-Dispersionen "ROTOVARIO" oder stabilisiertes leafing Goldbronzepigment-Pulver "ROTOFLEX" bzw. stabilisierte leafing Goldbronzepigment-Pellets "ROTOSAFE" der Fa. Eckart GmbH, D-90763 Fürth, Deutschland, sind aufgrund ihrer leafing-Eigenschaften für die Pigmentierung von Folienkonterapplikationen mit spiegelartigem Effekt nur bedingt geeignet. Die vorgenannten kupferhaltigen Metalleffektpigmente werden durch Trockenvermahlen von Kupfer oder Messinggrieß erhalten.

Auch der Einsatz von durch PVD-Verfahren hergestellten Messingpigmenten in Druckfarben ist insofern problematisch, da eine homogene Metallisierung der zwei Metalle (Kupfer und Zink) zur Erzielung eines gleichmäßigen Farbtones mit stark unterschiedlichen Verdampfungstemperaturen im Hochvakuum verfahrenstechnisch extrem schwierig ist. Zudem weisen die in der Herstellung relativ teuren PVD-Messingpigmente - im Gegensatz zu durch konventionelle Vermahlung hergestellten Messingpigmenten - weniger kompakte Schichten mit Dichten unterhalb der jeweiligen Materialdichten auf, und die gewünschten Goldfarbtöne (vor allem Reichgold) lassen sich nicht bei den gewünschten niedrigen Schichtdicken realisieren.

Die EP 1 529 084 B1 beschreibt Goldbronzepigmente, die durch PVD-Verfahren hergestellt werden können. Diese Pigmente sind aufgrund des aufwändigen Verfahrens sehr teuer. Zudem neigen diese Pigmente zu einer teilweisen Phasenseparation der Legierungsbestandteile, was ebenfalls unerwünschte Farbtonverschiebungen und nicht ausreichende Farbtonstabilitäten mit sich bringt.

Die GB 994,409 offenbart eine Lack- und Farbzusammensetzung, die kupferhaltige Pigmente und als Korrosionsinhibitor Benzotriazol enthält. Das Benzotriazol verhindert dabei eine durch Oxidation von Kupfer bedingte Verfärbung der Lack- bzw. Farbzusammensetzung.

Die US 6,398,861 B1 offenbart eine Metallpigmentzusammensetzung, die vermahlene Metallflocken enthält.

Die WO 2006/066825 A2 betrifft gegenüber Korrosion beständige plättchenförmige Metallpigmente,

Die DE 10315775 A1 betrifft Aluminiumeffektpigmente, die wenigstens teilweise mit Schmiermittel belegt sind.

Gemäß der Lehre der nichtvorveröffentlichten EP 08009699.3 werden kupferhaltige plättchenförmige Metalleffektpigmente mit verbesserten optischen Eigenschaften erhalten, wenn diese eine mit Rasterelektronenmikroskopie (REM) ermittelte Dickenverteilung in der Darstellung als Summendurchgangsverteilung mit einem $h_{50}$-Wert von 10 bis 50 nm und einen $h_{90}$-Wert von 20 bis 70 nm aufweisen.

Es hat sich nunmehr herausgestellt, dass die gemäß der Lehre der EP 08009699.3 hergestellten kupferhaltigen Metalleffektpigmente bei Kontakt mit Bindemitteln eines Beschichtungsmittels, beispielsweise Lack, Farbe, Druckfarbe, etc., zu einer unerwünschten Erhöhung der Viskosität des Lackes, der Farbe oder Druckfarbe, führen können. Dieser Viskositätsanstieg eines Beschichtungsmittels, beispielsweise einer Druckfarbe, der auch als Gelieren des Beschichtungsmittels, beispielsweise der Druckfarbe, bezeichnet werden kann, kann innerhalb von wenigen Stunden oder Tagen erfolgen. Eine derartig geliertes oder hochviskoses Beschichtungsmittel, beispielsweise Druckfarbe, kann nicht mehr bzw. nur unter Verlust der hochwertigen optischen Eigenschaften aufgebracht, beispielsweise verdruckt, werden. Das Beschichtungsmittel, beispielsweise eine Druckfarbe, kann, wenn es sehr hochviskos ist, nicht mehr unter Lösemittelzugabe dispergiert werden oder es muss eine sehr große Menge an Lösemittel zugegeben werden, die zu einer Beeinträchtigung der Qualität bei den optischen Eigenschaften des Beschichtungsmittels führt. Beispielsweise kann eine erhöhte Menge an Lösemittel im Falle einer Druckfarbe zu einer deutlichen Einbuße beim Farbtransfer während des Verdruckens führen.

Mithin besteht ein Bedarf an plättchenförmigen kupferhaltigen Metalleffektpigmenten, die hervorragende optische Eigenschaften, insbesondere bei

Folienkonterapplikationen, aufweisen und die keine unerwünschte Viskositätserhöhung nach Einbringung in ein Beschichtungsmittel hervorrufen.

Die der Erfindung zugrundeliegende Aufgabe wird durch Bereitstellung einer Mischung von durch Vermahlung erhaltenen plättchenförmigen kupferhaltigen Metalleffektpigmenten, die einen Kupfergehalt von 60 bis 100 Gew.%, bezogen auf den gesamten Metallgehalt, aufweisen, mit wenigstens einer weiteren Komponente, gelöst, wobei die kupferhaltigen Metalleffektpigmente eine mittlere Größe $d_{50}$ von 3 bis 50 $\mu$m und eine über Dickenauszählung mit Rasterelektronenmikroskopie (REM) ermittelte Dickenverteilung in der Darstellung als Summendurchgangsverteilung

a) mit einem $h_{50}$-Wert von 10 bis 100 nm,

b) mit einem $h_{50}$-Wert von 20 bis 150 nm

aufweisen und dass die wenigstens eine weitere Komponente ein Cellulosederivat, ausgewählt aus der Gruppe, die aus Alkylcellulose, Hydroxyalkylcellulose, Alkyl(hydroxyalkyl)cellulose und Mischenungen davon besteht, und/oder wenigstens ein Additiv mit antioxidativen und/oder radikalinhibierenden Eigenschaften ist. Der $h_{98}$-Wert liegt dabei vorzugsweise in einem Bereich von 21 bis 200 nm, weiter bevorzugt von 21 bis 80 nm.

Bevorzugte Weiterbildungen der Erfindung sind in den Unteransprüchen 2 bis 9 angegeben.

Des weiteren wird die der Erfindung zugrundeliegende Aufgabe auch durch Bereitstellung eines Beschichtungsmittels gelöst, wobei das Beschichtungsmittel eine Mischung nach einem der Ansprüche 1 bis 9 enthält und vorzugsweise eine Druckfarbe, ein Druckfarbenkonzentrat, ein Lack, ein Lackkonzentrat, eine Farbe oder ein Farbenkonzentrat ist.

Eine bevorzugte Weiterbildung des erfindungsgemäßen Beschichtungsmittels ist im Unteranspruch 11 angegeben.

Auch wird die der Erfindung zugrundeliegende Aufgabe durch Bereitstellung eines beschichteten Gegenstandes gelöst, wobei der beschichtete Gegenstand eine Mischung von kupferhaltigen Metalleffektpigmenten mit wenigstens einer Komponente nach einem der vorhergehenden Ansprüche 1 bis 9 oder ein Beschichtungsmittel nach einem der Ansprüche 10 bis 11 aufweist.

Gemäß einer bevorzugten Weiterbildung ist der Gegenstand transparent. Vorzugsweise ist der Gegenstand eine transparente Folie.

Die der Erfindung zugrundeliegende Aufgabe wird auch durch Bereitstellung eines Verfahrens zur Herstellung einer Mischung von kupferhaltigen Metalleffektpigmenten mit wenigstens einer Komponentenach einem der Ansprüche 1 bis 9 gelöst, wobei das Verfahren folgende Schritte umfasst:

(a) Vermahlen eines kupferhaltigen Metallgrießes mit einer Korngrößenverteilung mit einem $d_{Grieß,50}$ von 1 bis 180 $\mu$m und einem $d_{Grieß,90}$ von 2 bis 430 $\mu$m und einem Kupfergehalt von 60 bis 100 Gew.-%, bezogen auf den gesamten Metallgrieß zu plättchenförmigen Metalleffektpigmenten unter Verwendung eines Mahlwerks in Gegenwart von Schmiermitteln und Mahlkörpern und optional Lösemittel, wobei die so erhaltenen plättchenfömigen kupferhaltigen Metalleffektpigmente eine über Dickenauszählung mit Rasterelektronenmikroskopie (REM) ermittelte mittlere Dicke mit einem $h_{50}$-Wert von 10 bis 100 nm, vorzugsweise von 10 bis 50 nm, und einem $h_{90}$-Wert von 20 bis 150 nm, vorzugsweise von 20 bis 70 nm, aufweisen, und

(b) in Kontakt bringen der in Schritt (a) erhaltenen plättchenfömigen kupferhaltigen Metalleffektpigmente mit wenigstens einer Komponente, die ein Cellulosederivat, ausgewählt aus der Gruppe, die aus Alkylcellulose, Hydroxyalkylcellulose, Alkyl(hydroxyalkyl)cellulose und Mischungen davon besteht, und/oder wenigstens ein Additiv mit antioxidativen und/oder radikalinhibierenden Eigenschaften ist.

Die der Erfindung zugrundeliegende Aufgabe wird des weiteren durch die Verwendung einer Mischung von plättchenförmigen kupferhaltigen Metalleffektpigmenten nach einem der Ansprüche 1 bis 9 in einem, vorzugsweise in kompaktierter Form vorliegenden, Beschichtungsmittel gelöst.

Die der Erfindung zugrundeliegende Aufgabe wird des weiteren durch die Verwendung eines Cellulosederivats, ausgewählt aus der Gruppe, die aus Alkylcellulose, Hydroxyalkylcellulose, Alkyl(hydroxyalkyl)cellulose und Mischungen davon besteht, zur Stabilisierung von plättchenförmigen kupferhaltigen Metalleffektpigmenten, wie Messingeffektpigmenten, gelöst.

Die der Erfindung zugrundeliegende Aufgabe wird des weiteren durch die Verwendung eines Cellulosederivats, ausgewählt aus der Gruppe, die aus Alkylcellulose, Hydroxyalkylcellulose, Alkyl(hydroxyalkyl)cellulose und Mischungen davon besteht, zur Stabilisierung von Beschichtungszusammensetzungen gelöst, die plättchenförmige kupferhaltige Metalleffektpigmente, wie Messingeffektpigmente, enthalten.

Unter Stabilisierung wird verstanden, dass die plättchenförmigen kupferhaltigen Metalleffektpigmente gegenüber Oxidation geschützt sind bzw. die plättchenförmigen kupferhaltigen Metalleffektpigment-haltigen Beschichtungszusammensetzungen gegenüber einem Gelieren bzw einem unerwünschten Viskositätsanstieg geschützt sind.

Der erfindungsgemäß verwendete Begriff "Summendurchgangsverteilung" wird auch als "Summenhäufigkeitsverteilung" bezeichnet. Diese beiden Begriffe sind mithin austauschbar verwendbar, weshalb in der vorliegenden Anmeldung anstelle des Begriffs "Summendurchgangsverteilung" auch der Begriff "Summenhäufigkeitsverteilung" verwendet werden kann.

Der Begriff "plättchenförmiges kupferhaltiges Metalleffektpigment" und der Begriff "kupferhaltiges Metalleffektpigment" werden vorliegend austauschbar verwendet.

Die Erfinder haben festgestellt, dass die unerwünschte Viskositätserhöhung nach Einarbeitung der erfindungsgemäß verwendeten plättchenförmigen kupferhaltigen Metalleffektpigmente in ein Bindemittel-haltiges System, wie ein Beschichtungsmittel, auf ein Gelieren von Bindemittel zurückzuführen ist.

Die Erfinder vermuten, ohne dabei an diese Theorie gebunden sein zu wollen, dass das Gelieren des Bindemittels auf die Abgabe von einwertigen Kupferionen ($Cu^+$) aus den kupferhaltigen Metalleffektpigmenten zurückzuführen ist. Diese einwertigen Kupferionen gehen nachfolgend vermutlich unter Abgabe eines Elektrons in den stabileren zweiwertigen Zustand ($Cu^{2+}$) über. Hierdurch kommt es zur Reduktion von Bindemittel, wodurch es zu einer Vernetzung von Bindemittel und mithin zu einem Gelieren und der unerwünschten Viskositätserhöhung kommt.

Die Erfinder vermuten ferner, dass die erfindungsgemäß zu verwendenden kupferhaltigen Metalleffektpigmente aufgrund der großen spezifischen Oberfläche, d.h. Oberfläche pro Gewichtseinheit, eine sehr große Kontaktfläche mit umgebendem Bindemittel aufweisen. Die große Kontaktfläche führt vermutlich zu einer verstärkten Abgabe von Kupferionen in das Bindemittel. Die große spezifische Oberfläche ergibt sich durch die enge Dickenverteilung mit einem $h_{50}$-Wert aus einem Bereich von 10 bis 100 nm, insbesondere bei einem $h_{50}$-Wert aus einem Bereich von 10 bis 50 nm, und einem $h_{90}$-Wert aus einem Bereich von 20 bis 150, insbesondere bei einem $h_{90}$-Wert aus einem Bereich von 20 bis 70 nm, nm. Je geringer die Dicke der kupferhaltigen Metalleffektpigmente desto stärker geliert das Bindemittel. Die erfindungsgemäß zu verwendenden plättchenförmigen kupferhaltigen Metalleffektpigmente sind mithin Metalleffektpigmente, die eine äußerst geringe Dicke und mithin eine große spezifische Oberfläche aufweisen.

Insbesondere bei non-leafing plättchenförmigen kupferhaltigen Metalleffektpigmenten kommt es zu einem raschen Gelieren des Bindemittels, da non-leafing kupferhaltige Metalleffektpigmente vollständig, d.h. sowohl die untere als auch die obere Pigmentfläche, von Bindemittel umgeben sind. Bei non-leafing kupferhaltigen Metalleffektpigmenten können mithin Kupferionen über die untere und die obere Fläche angegeben werden.

Bei leafing kupferhaltigen Metalleffektpigmenten ist die Abgabe von Kupferionen geringer, da leafing Metalleffektpigmente aufschwimmen und mithin die obere Fläche nicht bzw. nur in geringem Umfang mit dem Bindemittel in Kontakt kommt. Sofern leafing kupferhaltige Metalleffektpigmente durch Zusatzstoffe, wie beispielsweise Zitronensäure, ein non-leafing Verhalten zeigen, tritt das Problem der verstärkten Abgabe von Kupferionen ebenfalls auf.

Der erfindungsgemäße Effekt, d.h. die Inhibierung des Gelierens eines kupferhaltige Metalleffektpigmente enthaltenen Beschichtungsmittels, zeigt sich auch bei kupferhaltigen plättchenförmigen PVD-Metalleffektpigmenten, die aufgrund einer geringen Metalleffektpigmentdicke ein hohes spezifisches Deckvermögen und mithin eine große mit Bindemittel in Kontakt kommende Oberfläche aufweisen. Insofern erstreckt sich die vorliegende Erfindung ebenfalls auf kupferhaltige PVD-Metalleffektpigmente, beispielsweise PVD-Messingeffektpigmente, sofern deren Dicken die in dieser Anmeldung angegebenen Dickenverteilungen aufweisen. Sämtliche Angaben in dieser Anmeldung beziehen sich mithin auf durch Vermahlung erhaltene kupferhaltige plättchenförmige Metalleffektpigmente als auch auf kupferhaltige PVD-Metalleffektpigmente, sofern deren Dickenverteilung(en) mit denen der vorliegenden Anmeldung übereinstimmen.

Durch das Gelieren von Bindemittel wird die Lagerstabilität eines Beschichtungsmittels, das die erfindungsgemäß zu verwendende Mischung von kupferhaltigen plättchenförmigen Metalleffektpigmenten enthält, signifikant verringert. Bei Kontakt der erfindungsgemäß zu verwendenden plättchenförmigen kupferhaltigen Metalleffektpigmente mit Bindemittel kann es bereits innerhalb von 24 Stunden zu einem Gelieren, mithin zu einer Viskositätserhöhung kommen.

Überraschenderweise kann ein Gelieren von Bindemittel unterdrückt, vorzugsweise verhindert, werden, wenn die erfindungsgemäß verwendeten plättchenförmigen kupferhaltigen Metalleffektpigmente wenigstens eine Komponente, die ein Cellulosederivat, ausgewählt aus der Gruppe, die aus Alkylcellulose, Hydroxyalkylcellulose, Alkyl(hydroxyalkyl)cellulose und Mischungen davon besteht, und/oder wenigstens ein Additiv mit antioxidativen und/oder radikalinhibierenden Eigenschaften aufweisen,

Unter Lagerstabilität versteht man eine gleichbleibende Viskosität bzw. einen geringen Anstieg der Viskosität und eine gleichbleibende Optik der Farbe bei sachgemäßer Lagerung innerhalb dieses Zeitraumes. Erfindungsgemäß beträgt die Lagerstabilität vorzugsweise wenigstens 6 oder 12 Monate bei Zimmertemperatur.

Die wenigstens eine Komponente, die ein Cellulosederivat, ausgewählt aus der Gruppe, die aus Alkylcellulose, Hydroxyalkylcellulose, Alkyl(hydroxyalkyl)cellulose und Mischungen davon besteht, wird vorzugsweise im Bereich von 0,1 Gew.-% bis 40 Gew.-%, bevorzugt von 0,5 bis 20 Gew.-%, vorzugsweise von 1 bis 10 Gew.%, weiter bevorzugt von 1 bis 5 Gew.-%, jeweils bezogen auf das Gesamtgewicht der Zubereitung, beispielsweise Farbe oder Druckfarbe, zugegeben.

Die wenigstens eine Komponente, die wenigstens ein Additiv mit antioxidativen und/oder radikalinhibierenden Eigenschaften ist, wird vorzugsweise im Bereich von 1 Gew.-% bis 15 Gew.-%, bevorzugt von 3 bis 10 Gew.-%, jeweils bezogen auf das Gewicht des eingesetzten Metalleffektpigmentes zugegeben.

Ein Additiv mit antioxidativen und/oder radikalinhibierenden Eigenschaften wirkt vornehmlich chemisch, indem das kupferhaltige Metalleffektpigment vor oxidativen Einflüssen durch Oxidation des Additivs mit antioxidativen Eigenschaften geschützt wird. Ein Additiv mit radikalinhibierenden Eigenschaften, beispielsweise ein Radikalfänger, wirkt, indem es von Kupferionen abgegebene Elektronen, beispielsweise bei Oxidation von einwertigem Kupfer ($Cu^*$) zu zweiwertigem Kupfer ($CU^{2+}$), abfängt.

Die Wirkungsweise des Additivs mit antioxidativen und/oder radikalinhibierenden Eigenschaften kann nicht eindeutig zugeordnet werden, da die antioxidativen und die radikalreduzierenden Eigenschaften voneinander getrennt oder auch gemeinsam zur Geltung kommen können.

Gemäß einer weiteren bevorzugten Ausführungsform der Erfindung liegt in der Mischung neben der wenigstens einen weiteren Komponente ein Korrosionsinhibitor vor.

Ein Korrosionsinhibitor wirkt vornehmlich physikalisch, indem es das kupferhaltige Metalleffektpigment gegenüber dem umgebenden Medium, beispielsweise Bindemittel eines Beschichtungsmittels, isoliert. Hierdurch wird zum einen eine Oxidation von Kupfer zu einwertigem Kupfer ($Cu^+$) unterdrückt, vorzugsweise verhindert. Zum anderen wird auch eine Abgabe von Kupferionen an das umgebende Medium unterdrückt, vorzugsweise verhindert.

Als sehr wirksam hat sich eine Kombination eines Cellulosederivats, ausgewählt aus der Gruppe, die aus Alkylcellulose, Hydroxyalkylcellulose, Alkyl(hydroxyalkyl)cellulose und Mischungen davon besteht, mit wenigstens einem Additiv mit antioxidativen und/oder radikafinhibierenden Eigenschaften zum Schutz von plättchenförmigen, kupferhaltigen Metalleffektpigmenten bzw. von plättchenförmigen, kupferhaltigen Metalleffektpigment-haltigen Beschichtungsmitteln herausgestellt.

Als sehr wirksam hat sich auch eine Kombination eines Cellulosederivats, ausgewählt aus der Gruppe, die aus Alkylcellulose, Hydroxyalkylcellulose, Alkyl(hydroxyalkyl)cellulose und Mischungen davon besteht, mit wenigstens einem Korrosionsinhibitor zum Schutz von plättchenförmigen, kupferhaltigen Metalleffektpigmenten bzw. von plättchenförmigen, kupferhaltigen Metalleffektpigment-haltigen Beschichtungsmitteln herausgestellt.

Des weiteren hat sich eine Kombination wenigstens eines Additivs mit antioxidativen und/oder radikalinhibierenden Eigenschaften mit wenigstens einem Korrosionsinhibitor zum Schutz von plättchenförmigen, kupferhaltigen Metalleffektpigmenten bzw. von plättchenförmigen, kupferhaltigen Metalleffektpigment-haltigen Beschichtungsmitteln herausgestellt.

Als sehr geeignet hat sich eine Kombination eines Cellulosederivats, ausgewählt aus der Gruppe, die aus Alkylcellulose, Hydroxyalkylcellulose, Alkyl(hydroxyalkyl)cellulose und Mischungen davon besteht, wenigstens eines Additivs mit antioxidativen und/oder radikalinhibierenden Eigenschaften und wenigstens einem Korrosionsinhibitor zum Schutz von plättchenförmigen, kupferhaltigen Metalleffektpigmenten bzw. von plättchenförmigen, kupferhaltigen Metalleffektpigment-haltigen Beschichtungsmitteln herausgestellt.

Die Lagerstabilität liegt bei einem erfindungsgemäßen Beschichtungsmittel vorzugsweise bei wenigstens 6 Monaten, vorzugsweise bei wenigstens 12 Monaten bei Zimmertemperatur, d.h. in einem Temperaturbereich von etwa 15 bis etwa 30°C, vorzugsweise von etwa 18 bis etwa 25 °C. Eine Lagerstabilität von 10 Wochen bei 40°C entspricht in etwa einer Lagerstabilität von 6 Monaten bei 25°C.

Unter Lagerstabilität wird verstanden, dass eine Beschichtungsmittel, z,B, eine Druckfarbe bei sachgemäßer Lagerung bei 25°C keine optische Veränderung erfährt und ungeachtet eines etwaigen, vorzugsweise geringfügigen, Viskositätsanstieg ohne weiteres aufrührbar und mithin bestimmungsgemäß verwendbar ist.

Ein lagerstabiles Beschichtungsmittel läßt sich ohne weiteres durch Rühren, gegebenenfalls unter Zugabe von organischem Lösemittel aufrühren, so dass die plättchenförmigen kupferhaltigen Metalleffektpigmente dispergiert sind und das Beschichtungsmittel beispielsweise als Farbe, Lack oder Druckfarbe bestimmungsgemäß verwendbar ist.

Gemäß einer bevorzugten Weiterbildung der Erfindung weisen die kupferhaltigen Metalleffektpigmente eine über Dickenauszählung mit Rasterelektronenmikroskopie (REM) ermittelte Dickenverteilung in der Darstellung als Summendurchgangsverteilung

    a) mit einem $h_{50}$-Wert von 10 bis 50 nm,
    b) mit einem $h_{90}$-Wert von 20 bis 70 nm auf.

Die erfindungsgemäß zu verwendenden plättchenförmigen kupferhaltigen Metalleffektpigmente besitzen aufgrund ihrer sehr geringen mittleren Dicke eine sehr hohe Deckkraft. Je dünner die mittlere Dicke der kupferhaltigen Metalleffektpigmente ist, desto höher ist die Deckkraft.

Als Deckkraft oder Deckvermögen eines Pigmentes wird üblicherweise die Abdeckung einer Fläche pro Gewichtseinheit an Pigmentmenge bezeichnet, Je dünner die mittlere Dicke der Pigmente ist, desto größer ist die durch das Pigment abgedeckte Fläche und mithin dessen Deckkraft.

Sehr dünne Metalleffektpigmente mit einer sehr engen Dickenverteilung stapeln sich gleichmäßiger im Anwendungsmedium als Metalleffektpigmente mit einer breiten Dickenverteilung. Bei den herkömmlichen Metalleffektpigmenten kann es leicht zu Ungleichmäßigkeiten in der Stapelung der Pigmente im Anwendungsmedium kommen. So können insbesondere sehr dicke Metalleffektpigmente als "Abstandshalter" wirken, welche die Orientierung der umgebenden bzw, benachbart liegenden Pigmente im Anwendungsmedium beeinträchtigt. Hiervon werden Glanz, Flop und unter Umständen das Deckvermögen der Metalleffektpigmente nachteilig beeinträchtigt. Dies wirkt sich insbesondere nachteilig in Druckanwendungen aus. Drucke haben im Vergleich zu Lackbeschichtungen (Coatings) eine wesentlich geringere Schichtdicke und einen geringeren Bindemittelanteil,

Die Bestimmung der exakten mittleren Dicke von plättchenförmigen Metallpigmenten ist schwierig. In der Praxis erfolgt die Pigmentdickenbestimmung über den Wasserbedeckungsgrad (Spreitung nach DIN 55923) oder durch Rasterelektronenmikroskopie (REM). Mit dem Wasserbedeckungsgrad lässt sich lediglich eine mittlere Dicke h der Pigmente berechnen, nicht aber die Dickenverteilung. Um auch die Dickenverteilung zu ermitteln, wurde im Rahmen dieser Erfindung die mittlere Dicke der erfindungsgemäß zu verwendenden Metallefffktpigmente mittels Rasterelektronenmikroskopie (REM) bestimmt. Bei dieser Methode sind so viele Teilchen zu vermessen, dass eine repräsentative statistische Auswertung vorgenommen werden kann. Üblicherweise werden etwa 50 bis 100 Teilchen vermessen.

Die Dickenverteilung wird zweckmäßigerweise in Form einer Summendurchgangsverteilung bzw. der Summenhäufigkeitsverteilung dargestellt. Als Mittelwert bietet sich der $h_{50}$-Wert der Summendurchgangsverteilung bzw. der Summenhäufigkeitsverteilung an. Ein Maß für den Grobanteil ist der $h_{90}$-Wert. Er besagt, dass 90 % aller Pigmentteilchen eine Dicke gleich diesem Wert und/oder unterhalb dieses Wertes besitzen. Entsprechend besagt beispielsweise ein $h_{98}$-Wert, dass 98 % aller Pigmentteilchen eine Dicke gleich diesem Wert und/oder unterhalb dieses Wertes besitzen. In analoger Weise ist der $h_{10}$-Wert ein Maß für den Feinanteil der Dickenverteilung, der besagt, dass 10 % aller Pigmentteilchen eine Dicke gleich diesem Wert und/oder unterhalb dieses Wertes besitzen.

Diese Werte können rechnerisch aus einer Liste der gemessenen Einzelwerte ermittelt werden, so z.B. mit Hilfe der "Quantil"-Funktion in einer Excel-Darstellung.

Die Ermittlung der Dicken der einzelnen Pigmente mittels REM erfolgt nach der in der DE 103 15 775 A1 beschriebenen Methode.

Im Ergebnis der Dickenauszählung mit Rasterelektronenmikroskopie ($h_{50}$-Wert der Summendurchgangsverteilung bzw. der Summenhäufigkeitsverteilung) wurde für die erfindungsgemäß zu verwendenden kupferhaltigen Metalleffektpigmente wie beispielsweise Goldbronzepigmente eine mittlere Dicke $h_{50}$ von 10 bis 100, weiter bevorzugt 10 bis 50 nm, bevorzugt von 15 bis 45 nm, besonders bevorzugt von 15 bis 40 nm und ganz besonders bevorzugt von 20 bis 35 nm, ermittelt.

Unterhalb einer mittleren Dicke $h_{50}$ von 10 nm werden die resultierenden Farbtöne der kupferhaltigen Metalleffektpigmente zu dunkel, was auf eine Minderung des Reflexionsvermögens unter Beibehaltung der hohen Absorptionseigenschaften des Kupfers bzw. Messings zurückzuführen ist. Ebenso vermindert sich aufgrund der zunehmenden Transparenz der kupferhaltigen Metalleffektpigmente die Deckkraft und es können sich unerwünschte Farbtonverschiebungen einstellen.

Oberhalb einer mittleren Dicke $h_{50}$ von 50 nm waren bei den erfindungsgemäß zu verwendenden kupferhaltigen Metalleffektpigmenten vorteilhafte optischen Eigenschaften nur noch in stark abgeschwächter Form vorhanden.

Außerhalb eines Bereiches mit einer mittleren Dicke $h_{50}$ von 10 bis 50 nm werden kupferfarbene Metalleffektpigmente erhalten, die goldfarben sind, jedoch keinen Spiegeleffekt mehr aufweisen. Diese Pigmente werden vorzugsweise bei Anwendungen verwendet, bei denen kein Spiegelglanz erwünscht oder erforderllich ist, wie beispielsweise bei Aufbringung einer Druckfarbe auf einem saugfähigen Untergrund, beispielsweise auf Papier.

Weiterhin weisen die erfindungsgemäß zu verwendenden kupferhaltigen Metalleffektpigmente einen über Dickenauszählung mit Rasterelektronenmikroskopie (REM) ermittelte Dickenverteilung mit einem $h_{90}$-Wert von 20 bis 150 nm, vorzugsweise von 20 nm bis 80 nm, weiter vorzugsweise von 20 nm bis 70 nm, bevorzugt von 20 bis 60 nm, weiter bevorzugt von 21 bis 50 nm und besonders bevorzugt von 22 bis 40 nm, auf.

Oberhalb eines $h_{90}$-Wertes von 70 nm waren die vorteilhaften Eigenschaften der erfindungsgemäß zu verwendenden Metalleffektpigmente nicht mehr zu beobachten. Insbesondere konnte ein klarer Spiegel bei einer Folienkonterapplikation (mit sehr guter Abbildeschärfe) nicht mehr festgestellt werden.

Plättchenförmige kupferhaltige Metalleffektpigmente mit einem $h_{90}$-Wert von unter 20 nm konnten bislang nicht mittels Vermahlung hergestellt werden.

Es wird vermutet, dass die vorteilhaften optischen Eigenschaften der erfindungsgemäß zu verwendenden Metalleffektpigmente auf einer sehr geringen Dicke aller Pigmente in der Pigmentdickenverteilung beruhen. Deshalb sollte der $h_{98}$-Wert bevorzugt im Bereich von 21 nm bis 200 nm, bevorzugt von 21 nm bis 90 nm, weiter bevorzugt von 21 bis unter 80 nm, besonders bevorzugt von 24 bis 70 nm und ganz besonders bevorzugt von 25 bis 60 nm liegen.

Vorteilhaft bewirken die geringen Dicken der erfindungsgemäß zu verwendenden kupferhaltigen Metalleffektpigmente eine sehr gute Orientierung der Pigmente im Anwendungsmedium, beispielsweise in einer Druckfarbe, insbesondere in einer Tief- und Flexodruckfarbe zur Herstellung von goldfarbigen Folienkonterapplikationen.

Es wird vermutet, dass ab einer bestimmten Plättchendicke diese derart flexibel werden, dass sie sich perfekt an den Untergrund anschmiegen. Dieser Effekt ist von PVD-Aluminiumpigmenten seit langem bekannt und wird insbesondere in den Folienkonterapplikationen ausgenutzt.

Bei einer weiterhin bevorzugten Ausführungsform der Erfindung weisen die erfindungsgemäß zu verwendenden Metalleffektpigmente einen $h_{10}$-Wert der Dickenverteilung im Bereich von 8 bis 50 nm, vorzugsweise von 8 bis 25 nm und besonders bevorzugt von 10 bis 20 nm auf. Unterhalb eines $h_{10}$-Wertes von 8 nm sind die Pigmente zu dünn, was zu verschlechterten optischen Eigenschaften führt. Bei einem $h_{10}$-Wert oberhalb von 25 nm weisen die kupferhaltigen Metalleffektpigmente keinen Spiegeleffekt mehr auf, sondern lediglich ein goldfarbenes Erscheinungsbild.

Weiterhin weisen die erfindungsgemäß zu verwendenden Metalleffektpigmente eine über Dickenauszählung mit Rasterelektronenmikroskopie (REM) ermittelte relative Breite der Dickenverteilung $\Delta h$, welche anhand der entspre-

chenden Summendurchgangskurve der relativen Häufigkeit bzw. Summenhäufigkeitsverteilung der relativen Häufigkeit nach der Formel

$$\Delta h = 100 \times (h_{90} - h_{10}) / h_{50}$$

berechnet wird, von 30 bis 100%, vorzugsweise von 30% bis 90%, bevorzugt von 35 bis 85 % und besonders bevorzugt von 40 bis 80%, auf.

Aufgrund der in überraschender Weise PVD-Metalleffektpigmenten ähnlichen, engen Dickenverteilung der durch Nassvermahlung hergestellten, erfindungsgemäß zu verwendenden Pigmente ähneln diese in ihren optischen Eigenschaften PVD-Pigmenten, sind jedoch wesentlicher kostengünstiger und mit zufriedenstellender Farbtonstabilität herzustellen.

In der Längsausdehnung unterscheiden sich die erfindungsgemäß zu verwendenden, insbesondere durch Nassvermahlung von Kupfer- oder Messinggrieß hergestellten Metalleffektpigmente nicht grundsätzlich von kommerziell gehandelten, durch Trockenvermahlung von Kupfer- oder Messinggrieß hergestellten Goldbronzepigmenten. Im Einzelnen hängen die Pigmentgrößen vom Einsatzzweck ab.

Die erfindungsgemäß zu verwendenden kupferhaltigen Metalleffektpigmente weisen eine mittlere Größe $d_{50}$ von 3 bis 50 $\mu$m, bevorzugt von 4 bis 30 $\mu$m, besonders bevorzugt von 5 bis 20 $\mu$m und ganz besonders bevorzugt von 6 bis 15 $\mu$m, auf.

Die Längsausdehnung d (Durchmesser) wird in Laserbeugungsexperimenten auf Grundlage der Fraunhofer- und/oder der Miebeugungstheorie bestimmt. Der Auswertung der Beugungsdaten liegt ein Modell zugrunde, welches auf den Durchmesser einer Äquivalentkugel abzielt. Daher werden keine Absolutwerte erhalten, jedoch haben sich die gemessenen Durchmesser als verlässliche Relativwerte in der Beschreibung der Größencharakteristik von plättchenförmigen Metallpigmenten durchgesetzt.

Der $d_{50}$-Wert der Pigmentlänge entspricht dabei 50% der Summendurchgangsverteilungskurve bzw. Summenhäufigkeitsverteilung, gemessen und ausgewertet in Form einer Volumenverteilung von Äquivalentkugeln.

Völlig überraschend wurde festgestellt, dass die mit den kupferhaltigen Metalleffektpigmenten, die einen $h_{50}$-Wert im Bereich von 10 bis 50 nm und einen $h_{90}$-Wert im Bereich von 20 bis 70 nm aufweisen, pigmentierten Beschichtungszusammensetzungen bei "Folienkonterapplikationen" einen goldfarbenen und spiegelartigen Effekt aufweisen, der mit herkömmlichen, durch Trockenvermahlung hergestellten kupferhaltigen Metalleffektpigmenten bisher nicht realisiert werden konnte.

Unter einer "Folienkonterapplikation" wird verstanden, dass eine mit Metalleffektpigmenten pigmentierte Druckfarbe auf eine transparente Folie aufgedruckt wird. Der auf der Folie ausgehärtete Druck ergibt bei Verwendung der erfindungsgemäß zu verwendenden kupferhaltigen Metalleffektpigmente einen goldfarbenen (goldfarbigen) spiegelartigen Effekt in der Konteransicht. Dieser Spiegeleffekt entsteht dadurch, dass sich wenigstens ein Teil der erfindungsgemäßen kupferhaltigen Metalleffektpigmente aufgrund ihrer geringen Dicke und ihrer engen Dickenverteilung vorzugsweise direkt an der Folienoberfläche ausrichten. Der an oder entlang der Folienoberfläche angeordnete Anteil der erfindungsgemäßen kupferhaltigen Metalleffektpigmente bewirkt den außerordentlichen Spiegelglanz. Die weiteren in dem Applikationsmedium, beispielsweise Druckfarbe, angeordneten erfindungsgemäßen kupferhaltigen Metalleffektpigmente sind wesentlich für die Deckung, d.h. Opazität.

Die erfindungsgemäß zu verwendenden plättchenförmigen kupferhaltigen Metalleffektpigmente sind Metalleffektpigmente, die einen Kupfergehalt von wenigstens 60 Gew.%, bezogen auf den gesamten Metallgehalt der Pigmente, aufweisen.

Die erfindungsgemäß zu verwendenden kupferhaltigen Metalleffektpigmente umfassen insbesondere Kupferpigmente sowie Zink und Kupfer enthaltende Messingpigmente (Goldbronzen).

Die Kupfereffektpigmente weisen vorzugsweise einen Kupfergehalt von 98 bis 100 Gew.-%, und bevorzugt von 99 bis 99,999 Gew.-%, jeweils bezogen auf den Gesamtmetallgehalt der Pigmente. Es versteht sich von selbst, dass der Fachmann bei der Angabe "100 Gew.-%" Kupfer auch gegebenenfalls in Spurenmengen enthaltene Fremdmetalle mitliest.

Die Messingeffektpigmente, üblicherweise als "Goldbronzen" bezeichnet, weisen bevorzugt einen Kupfergehalt von 70 bis weniger als 100 Gew.-%, vorzugsweise 75 bis 90 Gew.-%, auf. Der Zinkgehalt liegt vorzugsweise entsprechend zwischen 30 und 10 Gew.-%, wobei gegebenenfalls bis zu 2 Gew.-%, bevorzugt unter 1 Gew.-% Verunreinigungen anderer Metalle vorhanden sein können. Bei Messingeffektpigmenten oder Goldbronzeeffektpigmenten wird der Farbton durch das Kupfer-Zink-Verhältnis der Legierung bestimmt. Goldbronzeeffektpigmente werden in charakteristischen Naturfarbtönen als "Bleichgold" mit einem Kupferanteil von ca. 90 Gew.-% und Rest 10 Gew.-% Zink, als "Reichbleichgold" mit einem Kupferanteil von ca. 85 Gew.-% Kupfer und Rest 15 Gew.-% Zink sowie als "Reichgold" mit einem Kupferanteil von ca. 70 Gew.-% und Rest 30 Gew.-% Zink, kommerziell gehandelt. Die Angabe in Gew.-% bezieht sich hierbei auf den Gesamtmetallgehalt des Pigmentes.

Bei einer bevorzugten Ausführungsform enthalten die Messingeffektpigmente eine "Verunreinigung" mit beispielsweise 0,1 bis 2 Gew.-%, vorzugsweise 0,5 bis 2 Gew.-%, Aluminium, bezogen auf den Gesamtmetallgehalt des Metalleffektpigments. Derartige Legierungen haben sich als korrosionsstabiler, verglichen mit ausschließlich Kupfer und Zink enthaltende Messingeffektpigmente erwiesen.

Die erfindungsgemäß zu verwendenden plättchenförmigen kupferhaltigen Metalleffektpigmente können dabei ein leafing-Verhalten (leafing-Pigmente) oder ein non-leafing-Verhalten (non-leafing-Pigmente) aufweisen.

Unter non-leafing-Metalleffektpigmenten wird erfindungsgemäß verstanden, dass sich die Metalleffektpigmente überwiegend in einem Applikationsmedium, beispielsweise einer Druckfarbe, Farbe oder einem Lack, anordnen, so dass die Metalleffektpigmente vollständig von dem Applikationsmedium umgeben sind. Über die Dicke eines aufgebrachten Films eines Applikationsmediums sind die non-leafing-Metalleffektpigmente mithin verteilt, in weitgehend planparalleler Anordnung zur Oberfläche des Untergrundes, angeordnet.

Unter leafing-Metalleffektpigmenten wird erfindungsgemäß verstanden, dass sich die Metalleffektpigmente überwiegend an einer Grenzfläche (Oberfläche) oder in der Nähe einer Grenzfläche eines Applikationsmediums, beispielsweise einer Druckfarbe, Farbe oder einem Lack, anordnen, so dass die Metalleffektpigmente nicht vollständig von dem Applikationsmedium umgeben sind, sondern der Kontakt zwischen Metalleffektpigment und Applikationsmedium überwiegend nur über eine Fläche des Metalleffektpigmentes erfolgt.

Das non-leafing-Verhalten von kupferhaltigen Metalleffektpigmenten kann auch durch Zugabe von Zusatzstoffen, wie beispielsweise Zitronensäure, induziert werden. So können ursprünglich leafing kupferhaltige Metalleffektpigmente zu non-leafing Metalleffektpigmenten werden.

Als treibende Kraft für eine planparallele Orientierung von Metalleffektpigmenten ist, neben der grenzflächenchemischen Unverträglichkeit der Pigmente zum Bindemittelsystem, der Formfaktor ein weiteres wichtiges Charakteristikum für die Eigenschaften der erfindungsgemäß zu verwendenden kupferhaltigen Metalleffektpigmente.

Unter dem Formfaktor f versteht man das Verhältnis des Mittelwertes der Längsausdehnung zur mittleren Dicke der Pigmentplättchen.

Der dimensionslose Formfaktor f in dieser Erfindung ist definiert als:

$$f = 1000 * \frac{d_{50}(\mu m)}{h_{50(nm)}}$$

Die erfindungsgemäß zu verwendenden kupferhaltigen Metalleffektpigmente, beispielsweise Goldbronzepigmente, weisen vorzugsweise einen Formfaktor f von 30 bis 5000, weiter bevorzugt von 150 bis 3.000 auf. Bevorzugt sind die erfindungsgemäßen Pigmente durch einen Formfaktor f von 250 bis 2.500, weiter bevorzugt von 300 bis 1.000 und besonders bevorzugt von 325 bis 600, gekennzeichnet.

Bei Drucken sind die Bindemittelanteile und die Schichtdicken generell sehr viel niedriger als in Lacken. Dies trifft vor allem für Tiefdruckfarben zu. Mit kommerziell gehandelten Goldbronzepigmenten pigmentierte Tiefdruckfarben weisen einen Festkörpergehalt von ca. 40 Gew.-% auf. Druckfilme hieraus weisen eine Nassfilmschichtdicke von ca, 3 bis 6 μm und eine Trockenfilmschichtdicke von ca. 1,5 bis 3 μm auf. Im Fall von mit PVD-Pigmenten pigmentierten Tiefdruckfarben liegen die Festkörperanteile bei ca.5 bis 20 Gew.-% der gesamten Tiefdruckfarbe. Damit gehen Trockenfilmschichtdicken von nur 0,5 bis 1,5 μm einher. Bei diesen äußerst geringen Schichtdicken ist eine weitgehend gleichförmige planparallele Orientierung der Metallpigmente notwendig. Diese konnte bisher nur mit PVD-Metalleffektpigmenten erreicht werden.

Mit den erfindungsgemäß zu verwendenden kupferhaltigen Metalleffektpigmenten pigmentierte Druckanwendungen, insbesondere Folienkonterapplikationen, weisen aufgrund der geringen mittleren Teilchendicke und der engen Teilchendickenverteifung der verwendeten Metalleffektpigmente vergleichbare optische Effekte (bzgl. Glanz / Spiegel) wie bei mit herkömmlichen PVD-Metalleffektpigmenten pigmentierte Druckanwendungen auf.

Die in der erfindungsgemäßen Mischung vorzugsweise verwendeten plättchenförmigen kupferhaltige Metalleffektpigmente sind durch Vermahlung mit Mahlkörpern erhaltene Metalleffektpigmente. Diese unter Verwendung von Mahlkörpern erhaltene Metalleffektpigmente weisen im Unterschied zu PVD-Metalleffektpigmenten keine absolut plane Oberfläche, sondern eine Vertiefungen und Erhöhungen aufweisende Oberfläche auf. Des weiteren unterscheidet sich der Randbereich von durch PVD-Verfahren erhaltenen und durch Vermahlung erhaltenen Metalleffektpigmente signifikant. Bei PVD-Pigmenten weist der Randbereich relativ gerade Bruchkanten auf. Der Randbereich von durch Vermahlung bzw. Verformung erhaltenen kupferhaltigen Metalleffektpigmenten ist irregulär geformt oder ausgefranst und weist in der Regel auch Risse oder Spalten auf.

Gemäß einer weiteren bevorzugten Ausführungsform ist das wenigstens eine Additiv ein Antioxidans und/oder Radikalfänger.

Weiterhin ist bevorzugt, dass das wenigstens eine Additiv eine Additivmischung ist, die wenigstens zwei verschiedene Additive, die aus der Gruppe, die aus Antioxidans und/oder Radikalfänger und Korrosionsinhibitor besteht, ausgewählt wird, umfasst.

Mithin umfasst die bei der vorliegenden Erfindung verwendete Additivmischung vorzugsweise folgende Kombinationen:

- Antioxidans und Korrosionsinhibitor;
- Radikalfänger und Korrosionsinhibitor;
- Antioxidans und Radikalfänger;
- Antioxidans, Radikalfänger und Korrosionsinhibitor,

die jeweils optional in Verbindung mit einem Cellulosederivat, ausgewählt aus der Gruppe, die aus Alkylcellulose, Hydroxyalkylcellulose,. Alkyl(hydroxyalkyl)cellulose und Mischungen davon besteht, verwendet werden.

Gemäß einer bevorzugten Variante der Erfindung wird der Korrosionsinhibitor, der auch als passivierender Inhibitor bezeichnet wird, aus der Gruppe, die aus Fettsäuren, Carbonsäurederivaten, organische Phosphaten und Phosphonaten und deren Estern, organisch funktionalisierten Silanen, aliphatischen oder cyclischen Aminen, aliphatischen und aromatischen Nitroverbindungen, Sauerstoff-, Schwefel- oder Stickstoff enthaltenden Heterocyclen, Schwefel-/Stickstoffverbindungen höherer Ketone, Aldehyde und Alkohole, Thiole, ß-Diketone, ß-Ketoester und deren Gemischen besteht, ausgewählt.

Gemäß einer bevorzugten Ausführungsform sind Carbonsäurederivate als Korrosionsinhibitoren besonders bevorzugt. Gemäß einer äußerst bevorzugten Variante sind die Carbonsäurederivate ein Teilester einer Dicarbonsäure, Tricarbonsäure, Tetracarbonsäure oder einer Mischung derselben.

Bei den bevorzugten Dicarbonsäuren handelt es sich um Dicarbonsäuren mit 3 bis 20 C-Atomen, vorzugsweise 4 bis 8 C-Atomen, die vorzugsweise als Halbester vorliegen.

Vorzugsweise werden die Dicarbonsäuren aus der Gruppe ausgewählt, die aus Malonsäure, Bernsteinsäure, Glutarsäure, Adipinsäure, Pimelinsäure, Suberinsäure, Azelainsäure, Sebacinsäure, Maleinsäure, Fumarsäure, Sorbinsäure, Phthalsäure, Terephthalsäure, Dodecandisäure, Tetradecandisäure, Hexadecandisäure und Mischungen davon besteht. Vorzugsweise wird Bernsteinsäure verwendet.

Als sehr geeignet hat sich Bernsteinsäurehalbester erwiesen.

Bei den bevorzugten Tricarbonsäuren handelt es sich um Tricarbonsäuren mit 6 bis 20 C-Atomen, vorzugsweise 8 bis 12.

Vorzugsweise werden die Tricarbonsäuren aus der Gruppe ausgewählt, die aus 1,2,3, Propantricarbonsäure, Zitronensäure, Hemimellithsäure, Trimellithsäure, Trimesinsäure und Mischungen davon besteht.

Bei den bevorzugten Tetracarbonsäuren handelt es sich um Tetracarbonsäuren mit 10 bis 20 C-Atomen.

Beispielsweise kann Pyromellithsäure verwendet werden.

Die vorstehend genannten Carbonsäuren sind dabei vorzugsweise mit Alkoholen mit 1 bis 12 Kohlenstoffatomen teilverestert. Besonders bevorzugt sind Methanol, Ethanol, n-Propanol, Isopropanol, n-Butanol, sec-Butanol, Isobutanol, Amylalkohol, Isoamylalkohol, 1-Hexanol, 2-Hexanol, 3-Hexanol, cyclo-Hexanol und Mischungen davon.

Bevorzugt ist, dass die teilveresterten Di-, Tri- und/oder Tetracarbonsäuren über wenigstens eine freie Carboxylgruppe verfügen, über die eine Anbindung an die kupferhaltige Metalleffektpigmentoberfläche erfolgen kann.

Selbstverständlich können auch Mischungen von Di-, Tri- und Tetracarbonsäure, vorzugsweise der jeweiligen Teilester verwendet werden.

Erfindungsgemäß können weiterhin als Korrosionsinhibitoren verwendet werden:

- Organisch modifizierte Phosphonsäuren bzw. deren Ester der allgemeinen Formel $R-P(O)(OR_1)(OR_2)$, wobei: R = Alkyl, Aryl, Alkyl-aryl, Aryl-alkyl sowie Alkylether, insbesondere ethoxylierte Alkylether und $R_1$, $R_2$ = H, $C_nH_{2n+1}$, mit n = 1-6 ist, wobei Alkyl jeweils verzweigt oder unverzweigt sein kann. $R_1$ kann gleich oder unterschiedlich zu $R_2$ sein.

- Organisch modifizierte Phosphorsäuren und -ester der allgemeinen Formel $R-O-P(OR_1)(OR_2)$ mit R = Alkyl, Aryl, Alkyl-aryl, Aryl-alkyl sowie von Alkylether, insbesondere ethoxylierte Alkylether und $R_1$, $R_2$ = H, $C_nH_{2n+1}$, mit n = 1-6 ist, wobei Alkyl jeweils verzweigt oder unverzweigt sein kann.

Es können reine Phosphonsäuren oder -ester oder Phosphorsäuren oder -ester oder beliebige Mischungen derselben verwendet werden.

Weiterhin kann die passivierende Inhibitorschicht korrosionsinhibierende organisch funktionalisierte Silane, aliphatische oder cyclische Amine, aliphatische oder aromatische Nitroverbindungen oder Sauerstoff-, Schwefel- und/oder Stickstoff enthaltende Heterocyclen umfassen oder daraus bestehen. Hierbei werden gemäß einer bevorzugten Variante der Erfindung stickstoffhaltige heterocyclische Verbindungen verwendet. Besonders bevorzugt sind Triazole und ganz besonders bevorzugt sind Benzotriazole. Die Triazole und Benzotriazole können dabei unsubstituiert oder substituiert, beispielsweise mit einer oder mehreren Alkylgruppe mit 1 bis 12 C-Atomen, vorzugsweise 2 bis 6 C-Atomen, sein.

Weiterhin können beispielsweise Thioharnstoffderivate, Schwefel- und/oder Stickstoffverbindungen höherer Ketone, Aldehyde und Alkohole, wie Fettalkohole, oder Thiole oder Gemische derselben verwendet werden.

Die passivierende Inhibitorschicht kann auch aus den vorgenannten Substanzen bestehen. Bei Verwendung von Aminverbindungen weisen diese bevorzugt organische Reste mit mehr als 6 C-Atomen, vorzugsweise mit 8 bis 24 C-Atomen, weiter bevorzugt mit 10 bis 18 C-Atomen, auf. Bevorzugt werden diese Amine zusammen mit organischen Phosphonsäuren und/oder Phosphorsäureester, vorzugsweise wie oben spezifiziert, oder deren Gemische eingesetzt.

Gemäß einer weiteren bevorzugten Variante wird das Antioxidans und/oder der Radikalfänger aus der Gruppe, die aus Phenolen, Phenolderivaten, insbesondere Alkylhydroxyltoluol wie Butylhydroxytoluol, Dialkylhydroxytoluol vorzugsweise 2,6-Ditert-butyl-p-cresol, und Chinonen, Chinonderivaten, Nitrosoverbindungen, Nitronen, Vitamin C, Vitamin C-Derivaten, Viatmin E, Vitamin E-Derivaten und Mischungen davon besteht, ausgewählt.

Die gemäß der vorliegenden Erfindung bevorzugt verwendeten Additive gehören einerseits zur Klasse der Korrosionsinhibitoren, wie z.B. Carbonsäureester und -halbester und Triazolderivate, und anderseits zur Klasse der Antioxidantien und Radikalinhibitoren, wie z.B. Phenolderivate, Vitamin C und E oder Chinone bzw. Chinonderivate. Diese Additive haben sich als Zusatz zu Beschichtungsmitteln, insbesondere als Zusatz bei Druckfarben, als sehr geeignet erwiesen. Druckfarben, die die erfindungsgemäß zu verwendenden plättchenförmigen kupferhaltigen Metalleffektpigmente sowie wenigstens eines der vorgenannten Additive aufweisen, weisen eine signifikant verlängerte Lagerstabilität auf.

Besonders bevorzugt ist eine Kombination der vorgenannten Additive, da dann sowohl eine antioxidative und/oder radikalinhibierende als auch korrosionsinhibierende Wirkung vorliegt,

Gemäß einer weiteren bevorzugten Ausführungsform der Erfindung umhüllt der Korrosionsinhibitor das plättchenförmige kupferhaltige Metalleffektpigment. Bei dem Korrosionsinhibitor kann es sich um eine umhüllende anorganische, organischchemische oder anorganisch/organisch-chemische Beschichtung handeln.

Korrosionsschutzschichten, die bei den erfindungsgemäß zu verwendenden Metalleffektpigmenten einen besonders guten Korrosionsschutz gewährleisten, umfassen oder bestehen aus Siliziumoxid, bevorzugt Siliziumdioxid, Zirkoniumoxid, Ceroxid, Aluminiumoxid, polymerisierten Kunststoffharzen, Phosphaten, Phosphiten, Boraten oder Mischungen derselben.

Bevorzugt sind Siliciumdioxid-Schichten, wobei die Siliziumdioxid-Oberfläche vorzugsweise mit Silanen belegt ist

Die $SiO_2$-Schichten werden bevorzugt durch Sol-Gel-Verfahren mit durchschnittlichen Schichtdicken von 2 bis 150 nm und bevorzugt von 5 bis 40 nm in organischen Lösemitteln hergestellt.

Gemäß einer weiteren Variante der Erfindung können die kupferhaltigen Metalleffektpigmente eine Metalloxidschicht als Korrosionsschutz aufweisen, wobei das Metall der Metalloxidschicht von gleicher Art wie das Metall des Metalleffektpigmentes ist.

Derartige Oxidschichten sind auch von üblichen Kupfer- bzw. Goldbronzeeffektpigmenten bekannt.

Diese Oxidschichten werden durch sogenannte Feuerfärbungen erhalten. Aufgrund ihrer Eigenfarben und von Interferenzeffekten ergeben diese Metalloxidschichten in Abhängigkeit von ihrer Schichtdicke Effektpigmente unterschiedlichster Farbtöne im gelb-roten Farbbereich. Selbstverständlich spielt hierbei auch die Grundfarbe des Metalleffektpigmentes eine große Rolle.

Bei der Oxidation wird stets ein Teil des Metalls in das entsprechende Oxid umgewandelt. Insofern ist es bevorzugt, nicht die dünnsten erfindungsgemäß zu verwendenden Metalleffektpigmente zur Oxidation heran zu ziehen. Diese hätten nur noch einen sehr geringen bis gar keinen Metallgehalt mehr, was große Nachteile in Bezug auf ihre Deckkraft hätte. Daher werden zur Oxidation ("Feuerfärbung") vorzugsweise Metalleffektpigmente mit mittleren Dicken $h_{50}$ im Bereich von 25 bis 50 nm verwendet.

Bei der Feuerbehandlung von kupferhaltigen Metalleffektpigmenten wirkt Luftsauerstoff über einen bestimmten Zeitraum unter definierter Temperatur auf das kupferhaltige Metalleffektpigment ein, wodurch sich eine dünne Oxidschicht auf dem kupferhaltigen Metallplättchen ausbildet. Durch Interferenzreflexion werden interessante Farbnuancierungen hervorgerufen. Feuereingefärbte kupferhaltige Metalleffektpigmente werden u. a. in den Farbtönen Englischgrün-, Zitron-, Dukatengold- und Feuerrottönen kommerziell gehandelt.

Bei der Erfindung können mithin auch feuergefärbte und somit gegenüber Korrosion geschützte kupferhaltige Metalleffektpigmente verwendet werden.

Gemäß einer Variante der Erfindung enthält die Mischung Cellulosederivate, die aus der Gruppe, die aus Alkylcellulose, Hydroxyalkylcellulose, Alkyl(hydroxyalkyl)cellulose und Mischungen davon besteht, ausgewählt werden.

Die Alkylcellulose und/oder Hydroxyalkylcellulose und/oder Alkyl(hydroxyalkyl)cellulose weisen vorzugsweise einen oder mehrere Alkylrest(e) und/oder Hydroxyalkylrest(e) und/oder Alkyl(hydroxyalkyl)rest(e), der bzw. die unabhängig voneinander 1 bis 8, vorzugsweise 2 bis 6, C-Atome aufweist bzw. aufweisen, auf. Bei dem Alkylrest kann es sich auch um einen Cycloalkylrest Hierbei haben sich insbesondere Methylcellulose, Ethylcellulose, Propylcellulose, (Hydroxyethyl)cellulose, (Hydroxypropyl)methylcellulose, Ethyl(hydroxyethyl)cellulose, Benzylcellulose, Carboxymethylcellulose oder Mischungen als sehr geeignet herausgestellt.

Besonders bevorzugt wird Ethylcellulose verwendet. Es hat sich überraschend herausgestellt, dass Ethylcellulose aus bislang nicht verstandenen Gründen besonders stabilisierend wirkt. Somit erlaubt Ethylcellulose die Bereitstellung von lagerstabilen erfindungsgemäßen Mischungen bzw. von erfindungsgemäßen Beschichtungsmitteln.

Neben den vorgenannten Cellulosederivaten können selbstverständlich auch weitere Celluloseester, Celluloseether und Mischungen davon in der erfindungsgemäßen Mischung bzw. dem erfindungsgemäßen Beschichtungsmittel enthalten sein.

Als Celluloseester haben sich als sehr geeignet Celluloseactetat, Celluloseacetobutyrat, Cellulosepropionat, Celluloseacetopropionatoder Mischungen davon herausgestellt.

Eine erfindungsgemäße Mischung von plättchenförmigen kupferhaltigen Metalleffektpigmenten mit wenigstens einem Additiv mit antioxidativen und/oder radikalinhibierenden Eigenschaften und mit Celluloseether, insbesondere Ethylcellulose, ist eine besonders bevorzugte Ausführungsform der Erfindung. Diese Mischung eignet sich hervorragend zur direkten Einarbeitung in Farben, insbesondere Druckfarben oder Druckfarbenkonzentrate. Weiterhin ist bevorzugt, dass die vorgenannte erfindungsgemäße Mischung zusätzlich einen Korrosionsinhibitor enthält.

Die Verwendung von Ethylcellulose ermöglicht die Bereitstellung einer Mischung mit organischem Lösemittel bzw. organischem Lösemittelgemisch.

Ein organisches Lösemittelgemisch kann erfindungsgemäß neben organischem Lösemittel auch Wasser in einer Menge von 0 bis 10 Gew.%, vorzugsweise von 1 bis 8 Gew.-%, weiter bevorzugt von 2 bis 5 Gew.-%, jeweils bezogen auf das Gesamtgewicht an Lösemittel, enthalten. Äußerst bevorzugt beträgt der Gehalt an Wasser in dem organischen Lösemittel weniger als 2 Gew,%, bezogen auf das Gesamtgewicht an Lösemittel. Mithin werden vorzugsweise technische organische Lösemittel verwendet, die einen Restgehalt an Wasser aufweisen können.

Da insbesondere brillante Druckfarben lösemittelbasiert, d.h. organisches Lösemittel enthaltend, sind, ermöglicht die Verwendung von Ethylcellulose, das ein Bindemittel ist, die Bereitstellung eines direkt für die Herstellung von Druckfarben verwendbaren Vorproduktes oder Konzentrats. Die unter Verwendung von Ethylcellulose bereitgestellten Druckfarben weisen eine außerordentliche Stabilität auf, d.h. geringe Neigung zum Gelieren.

Gemäß einer weiter bevorzugten Ausführungsform umfasst die Mischung ein organisches Lösemittel oder organisches Lösemittelgemisch.

Geeignete Lösemittel sind Alkohole, beispielsweise Methanol, Ethanol, 1 -Propanol, 2-Propanol oder Mischungen davon. Es können auch substituierte Alkohole, wie beispielsweise Alkoxyalkohole verwendet werden, wobei der Alkoxyrest und der Alkoholrest unabhängig voneinander 1 bis 4 , vorzugsweise 2 bis 3, C-Atome aufweisen können. Als sehr geeignet haben sich Ethoxypropanol und/oder Methoxypropanol erwiesen. Als Lösemittel können auch Ester, vorzugsweise Carbonsäurealkylester, verwendet werden, wobei der Carbonsäurerest und der Alkylrest unabhängig voneinander 1 bis 4 , vorzugsweise 2 bis 3, C-Atome aufweisen können. Als sehr geeignet haben sich Ethylacetat, Isopropylacetat, n-Propylacetat und/oder n-Butylacetat.

Es können selbstverständlich auch Gemische verschiedener Lösemittel eingesetzt werden. Beispielsweise kann es sich um ein Gemisch aus Ethanol und Estern wie Ethylacetat oder n-Propylacetat handeln.

Gemäß einer bevorzugten Ausführungsform ist das Beschichtungsmittel eine Druckfarbe, ein Druckfarbenkonzentrat, ein Lack, ein Lackkonzentrat, eine Farbe oder ein Farbenkonzentrat.

Das erfindungsgemäße Beschichtungsmittel liegt dabei vorzugsweise in kompaktierter Form, vorzugsweise als Pellet, Granulat, Tabletten, Briketts und/oder Würstchen, vor. Gemäß einer weiteren bevorzugten Variante kann die Mischung gemäß einem der Ansprüche 1 bis 9 ebenfalls als kompaktierte Form, vorzugsweise als Pellet, Granulat, Tabletten, Briketts und/oder Würstchen, vorliegen. Insoweit gelten die nachstehenden Ausführungen sowohl für die erfindungsgemäße Mischung gemäß einem der Ansprüche 1 bis 9 als auch für das erfindungsgemäße Beschichtungsmittel gemäß einem der Ansprüche 10 bis 11.

Eine weitere bevorzugte Ausführungsform betrifft die Verwendung einer Mischung von plättchenförmigen kupferhaltigen Metalleffektpigmenten nach einem der Ansprüche 1 bis 9 in einem, vorzugsweise in kompaktierter Form vorliegenden, Beschichtungsmittel.

Gemäß einer bevorzugten Ausführungsform liegt die erfindungsgemäße Mischung bzw. das erfindungsgemäße Beschichtungsmittel als pastenförmiges Produkt vor.

Der Festkörpergehalt dieser die kupferhaltigen Metalleffektpigmente enthaltenden Pasten liegt vorzugsweise in einem Bereich von 30 bis 90 Gew.-%, bevorzugt 40 bis 75 Gew.-% und besonders bevorzugt 45 bis 70 Gew.-%, jeweils bezogen auf das Gesamtgewicht der Paste.

Gemäß einer bevorzugten Weiterbildung liegt die erfindungsgemäße Mischung bzw. liegt das erfindungsgemäße Beschichtungsmittel in einer lösemittelarmen oder lösemittelfreien Form vor. Weiter bevorzugt liegt die erfindungsgemäße Mischung bzw. liegt das erfindungsgemäße Beschichtungsmittel in einer staubarmen, vorzugsweise staubfreien. Darreichungsform vor.

Eine lösemittelarme oder lösemittelfreie Darreichungsform kann durch Trocknen einer lösemittelhaltigen erfindungsgemäßen Mischung gemäß einem der Ansprüche 1 bis 9 oder eines lösemittelhaltigen Beschichtungsmittels gemäß einem der Ansprüche 10 bis 11 erhalten werden, wodurch ein weitgehend trockenes oder trockenes Pulver, vorzugsweise ein stabilisiertes kupferhaltiges Metalleffektpigmentpulver, erhalten wird.

Das trockene oder getrocknete kupferhaltige Metalleffektpigmentpulver kann durch Zugabe von, vorzugsweise organischem, Lösemittel, vorzugsweise von weniger als 15 Gew.-%, weiter vorzugsweise von 1 bis 11 Gew.-%, noch weiter bevorzugt von weniger als 10 Gew.-%, noch weiter bevorzugt von 3 bis 8 Gew.-%, jeweils bezogen auf das Gesamtgewicht der Zubereitung, in einem geeigneten Homogenisator zu einem nicht staubenden Metallpulver, vorzugsweise in kompaktierter Form, weiterverarbeitet werden. Eine Kompaktierung kann durch Pelletieren, Granulieren, Tablettieren, Brikettieren, Extrudieren, etc. bewirkt werden.

Auch ist es selbstverständlich möglich, durch Filtration einer Zubereitung, beispielsweise einer Dispersion wie einer Paste zunächst einen Filterkuchen zu erhalten, diesen sodann zu trocknen und anschließend mit einem anderen Lösemittel wieder anzupasten (Umnetzen).

Überraschenderweise können die erfindungsgemäß zu verwendenden kupferhaltigen Metalleffektpigmente aber auch durch Versetzen eines Filterkuchens mit einer Dispersion eines Harzes in ein Granulat, Pellet, Brikett, Tabletten oder Würstchen überführt werden.

Diese Darreichungsformen besitzen die Vorteile, dass sie nicht stauben, eine leichte Dosierbarkeit aufweisen und hervorragend dispergierbar sind.

Die erfindungsgemäßen Mischungen können mithin äußerst vorteilhaft in kompaktierter Form, beispielsweise als Granulate, Pellets, Tabletten, Briketts, Würstchen, etc., mit hohen Gehalten an kupferhaltigem Metalleffektpigment, beispielsweise von 95 Gew.-% bis 35 Gew,-%, vorzugsweise von 85 Gew.-% bis 65 Gew.%, jeweils bezogen auf das Gesamtgewicht an kompaktierter Form, bereitgestellt werden. Die Restfeuchte, d.h. Gehalt an, vorzugsweise organischem, Lösemittel liegt dabei vorzugsweise in einem Bereich von 0 bis 15 Gew.-%, vorzugsweise von 1 bis 11 Gew.-%, weiter bevorzugt von 3 bis 8 Gew.-%, jeweils bezogen auf das Gesamtgewicht an kompaktierter Form.

Aufgrund der hohen spezifischen Oberfläche der erfindungsgemäß zu verwendenden kupferhaltigen Metalleffektpigmente müssen beispielsweise zu deren Kompaktierung, beispielsweise Pelletierung, Tablettierung, Brikettierung, Granulierung, etc., relativ große Mengen an Dispergierharz verwendet werden. Vorzugsweise werden 2 bis 50 Gew.-%, weiter bevorzugt 5 bis 30 Gew.-%, Harz, bezogen auf die Gesamtformulierung der kompaktierten Form, beispielsweise Pellets, Tabletten, Briketts, Würstchen, Granulat, etc., verwendet.

Zur Kompaktierung, beispielsweise Pelletierung, Tablettierung, Brikettierung, Granulierung, etc., kann eine Vielzahl von Dispergierharzen verwendet werden. Beispiele hierfür sind sowohl natürlich vorkommende wie auch synthetische Harze oder Mischungen davon. Sie umfassen beispielsweise Alkydharze, Carboxymethyl- und Carboxyethylcelluloseharze, Cellulose Acetat, Celluloseacetatpropionat (CAP) und Celluloseacetatbutyrat (CAB), Cumarol-Indenharze, Epoxidester, Epoxid-Meiamin und Epoxid-Phenol-Kondensate, Ethyl- und/oder Methylcellulose, Ethylhydroxyethylcellulose, Hydroxyethylcellulose, Hydroxypropylmethylcellulose, Keton- und/oder Maleinsäureharze, Kolophoniumharze, Melaminharze, Phenol- und/oder modifizierte Phenolharze, Polyacrylamid-, Polycarbonat-, Polyamid-, Polyester-, Polyether-, Polyurethan-, und/oder Vinylharze sowie Mischungen davon.

Unter diesen polymeren Harzen sind folgende Harze besonders geeignet: acrylatische Copolymere und/oder Acrylesterharze, Polyacrylonitril- und/oder Acrylonitrilcopolymerharze, Copolymere aus Butadien und Vinylidenchloride, Butadien/Styrol Copolymere, Methylacrylat- und/oder Methymethacrylatcopolymere; sowie Polybuten-, Polyisobutylen-, Polyvinylacetat-, Polyvinylakohol-, Polyvinylchlorid-, Polyvinylether-, Polyvinylpyrrolidon- und/oder Polystyrolharze. Weitere Copolymere beinhalten Styrol/Maleinsäureanhydrid- und/oder Styrol/Schellackharze, Vinyl-chlorid/Vinylacetat-, Vinylchlorid/Vinylether- und/oder Vinylchlorid/Vinylidenchloridharze sowie Mischungen davon.

Ferner können erfindungsgemäß auch natürlich vorkommende Harze wie Gummi Arabicum, Gutta Percha, Casein, Gelatine sowie Mischungen davon verwendet werden.

Bevorzugt sind Aldehydharze, wie die Laropalserie der BASF AG, Ludwigshafen. Weiterhin kommen Wachse als Bindermaterialien in Frage. Hier sind als Beispiele natürliche Wachse wie Bienenwachs, Candelilla-, Carnauba-, Montan- sowie Paraffinwachse oder Mischungen davon zu nennen. Ebenso kommen synthetische Wachse wie beispielsweise PE-Wachse in Betracht.

Besonders bevorzugt ist das Beschichtungsmittel eine Druckfarbe oder ein Druckfarbenkonzentrat.

Erfindungsgemäße Druckfarben auf Basis plättchenförmiger kupferhaltiger Metalleffektpigmente, wie oben spezifiziert, können vorteilhaft bei einer Vielzahl von Druckanwendungen, wie beispielsweise Tiefdruck oder Digitaldruck, verwendet werden. Mit den erfindungsgemäßen Druckfarben lassen sich brillante Goldeffekte auf hochwertigen Verpackungen oder Etiketten eines Produktes erzeugen die den Wert des Produktes steigern sollen.

Mit erfindungsgemäßen Druckfarben, die plättchenförmige kupferhaltige non-leafing-Effektpigmente enthalten, kann bei der sogenannten Konterapplikation, bei der eine transparente Folie mit der Druckfarbe bedruckt ist, ein nahezu perfekter Metallspiegel erzeugt werden. Dieser Metallspiegel wird sichtbar, wenn man die Applikation von der Folienseite, d.h. der von der bedruckten Fläche abgewandten Fläche der Folie, betrachtet.

Erfindungsgemäße Druckfarben, die plättchenförmige kupferhaltige leafing-Effektpigmente enthalten, können beim Oberflächendruck auf Papier und Karton, wie z.B. für Zigarettenverpackungen, Pralinenverpackungen oder Etiketten, verwendet werden.

Die erfindungsgemäßen lösemittelbasierenden Druckfarben enthalten Metalleffektpigment vorzugsweise in einer Konzentration zwischen 3 und 35 Gew.-%, weiter bevorzugt 5 bis 30 Gew.%, noch weiter bevorzugt, 10 bis 20 Gew.-%, wenigstens ein Bindemittel auf Polymerbasis vorzugsweise in einer Konzentration von 1 bis 40 %, vorzugsweise von 1 bis 30 Gew.-%, weiter bevorzugt von 2 bis 25 Gew.%, noch weiter bevorzugt von 2 bis 10 Gew.%, Lösemittel, jeweils bezogen auf das Gesamtgewicht der Druckfarbe, sowie von bis zu 15 Gew.-%, vorzugsweise von 0,1 bis 9 Gew.-%, weiter bevorzugt von 0, 2 bis 5 Gew.%, noch weiter bevorzugt von 0,3 bis 2,5 Gew.-%, der wenigstens einen weiteren Komponente, wobei sich die Angaben in Gew.-% in Bezug auf die wenigstens eine weitere Komponente auf das Gewicht an kupferhaltigem Metalleffektpigment beziehen, sowie optional weitere Hilfsstoffe.

Die vorliegende Erfindung betrifft auch Druckfarbenkonzentrate, die in ihrem Aufbau einer erfindungsgemäßenorganischen Lösemittel-basierten Druckfarbe ähneln, aber einen geringeren Anteil an Lösemittel besitzen. Um eine druckfertige erfindungsgemäße Druckfarbe zu erhalten, wird Lösemittel bis zur gewünschten Konsistenz bzw. Viskosität hinzugegeben. Druckfarbenkonzentrate bieten den Vorteil, dass in Abhängigkeit der Drucktechnik die jeweilige Viskosität durch Zugabe an Lösemittel eingestellt werden kann, mithin ein Druckfarbenkonzentrat für verschiedene Druckverfahren bzw. Druckmaschinen verwendet werden kann.

Die erfindungsgemäße Mischung kann besonders bevorzugt in Druckfarben oder Druckfarbenkonzentraten für den Tief-, Flexo-, Sieb- oder Digitaldruck eingesetzt werden.

Ein weiterer Gegenstand der Erfindung ist eine Druckfarbe, bevorzugt eine Tief-, Flexo- oder Sieb-, Digitaldruckfarbe, die die erfindungsgemäße Mischung enthält oder ein Druckfarbenkonzentrat, das die erfindungsgemäße Mischung enthält.

Diese erfindungsgemäße Druckfarbe oder dieses erfindungsgemäße Druckfarbenkonzentrat zeigt eine signifikante verbesserte Lagerstabilität im Vergleich zu Druckfarben oder Druckfarbenkonzentraten auf Basis der spezifizierten plättchenförmigen kupferhaltigen Metalleffektpigmente ohne die wenigstens eine weitere Komponente gemäß der vorliegenden Erfindung.

So zeigt z.B. eine Druckfarbe mit den in Anspruch 1 spezifizierten plättchenförmigen kupferhaltigen Metalleffektpigmenten ohne die wenigstens eine weitere Komponente und Polyvinylbutyral (PVB) als Bindemittel einen deutlichen Viskositätsanstieg innerhalb der ersten Stunden nach der Produktion und ein Gelieren innerhalb von 24 Stunden auf.

Die wenigstens eine weitere Komponente, die ein Cellulosederivat, ausgewählt aus der Gruppe, die aus Alkylcellulose, Hydroxyalkylcellulose, Alkyl(hydroxyalkyl)cellulose und Mischungen davon besteht, und/oder wenigstens ein Additiv mit antioxidativen und/oder radikalinhibierenden Eigenschaften ist, wird in einer Menge von bis zu 15 Gew.%, vorzugsweise von 0,1 bis 9 Gew.-%, weiter bevorzugt von 0,2 bis 5 Gew.%, noch weiter bevorzugt 0,3 und 2,5 Gew.-%, jeweils bezogen auf das Gewicht an kupferhaltigem Metalleffektpigment, der erfindungsgemäßen Mischung oder dem erfindungsgemäßen Beschichtungsmittel zugesetzt.

Eine Kombination von wenigstens einem Additiv mit antioxidativen und/oder radikalinhibierenden Eigenschaften und einem Cellulosederivat, ausgewählt aus der Gruppe, die aus Alkylcellulose, Hydroxyalkylcellulose, Alkyl(hydroxyalkyl)cellulose und Mischungen davon besteht, und vorzugsweise einem Korrosionsinhibitor ist besonders bevorzugt und zeigt eine deutliche Stabilisierung des erfindungsgemäßen Beschichtungsmittel, insbesondere von Druckfarben.

Die erfindungsgemäßen Druckfarben enthalten, vorzugsweise organisches, Lösemittel bzw. Lösemittelgemische. Diese dienen unter anderem zum Lösen der Bindemittel, aber auch zur Einstellung wichtiger Anwendungseigenschaften dieser Druckfarben, wie beispielsweise der Viskosität oder der Trocknungsgeschwindigkeit. Für Tief- und Flexodruckfarben eingesetzte Lösemittel umfassen insbesondere niedrig siedende Lösemittel. Der Siedepunkt beträgt im Regelfall nicht mehr als 140°C. Höher siedende Lösemittel werden nur in kleineren Mengen zur Einstellung der Trocknungsgeschwindigkeit eingesetzt.

Beispiele besonders geeigneter Lösemittel für Flüssigdruckfarben umfassen Ethanol, 1 -Propanol oder 2-Propanol, substituierte Alkohole, beispielsweise Ethoxypropanol oder Methoxypropanol oder Ester, beispielsweise Ethylacetat, Isopropylacetat, n-Propyl- oder n-Butylacetat. Es können selbstverständlich auch Gemische verschiedener Lösemittel eingesetzt werden. Beispielsweise kann es sich um ein Gemisch aus Ethanol und Estern wie Ethylacetat oder n- Propylacetat handeln.

In einer erfindungsgemäßen Druckfarbe sind üblicherweise 10 bis 80 Gew.-% Lösemittel, bezogen auf das Gesamtgewicht der Druckfarbe, enthalten. Für das Drucken mit Flexodruckfarben ist erfindungsgemäß bevorzugt, dass der Anteil der Ester am Gesamtlösemittel ca. 20 bis 25 Gew.-% nicht überschreitet.

Für erfindungsgemäße Druckfarben erweist sich ein Anteil an, vorzugsweise organischem, Lösemittel oder eines Lösemittelgemisches im Bereich von 60 bis 80 Gew.-%, bezogen auf das Gesamtgewicht der Druckfarbe, als besonders vorteilhaft.

Druckfarbenkonzentrate unterscheiden sich von Druckfarben im Hinblick auf den Lösemittelanteil. Der, vorzugsweise organische, Lösemittelanteil in den erfindungsgemäßen Druckfarbenkonzentraten liegt vorzugsweise in einem Bereich 10 - 50 Gew.-%, weiter bevorzugt von 12 bis 30 Gew.-%, noch weiter bevorzugt von 15 bis 25 Gew.-%,

jeweils bezogen auf das Gesamtgewichts des Druckfarbenkonzentrats. Um eine gebrauchsfertige Druckfarbe zu erhalten, werden üblicherweise 40 - 85 Gew.-%, vorzugsweise organisches, Lösemittel, bezogen auf das Gesamtgewicht des Druckfarbenkonzentrats, hinzugefügt.

Als Bindemittel für die erfindungsgemäßen Beschichtungsmittel, insbesondere Druckfarben, können prinzipiell die für Flüssig-Druckfarben üblichen Bindemittel eingesetzt werden, die je nach dem gewünschten Anwendungszweck und den gewünschten Eigenschaften ausgewählt werden.

Beispiele geeigneter Bindemittel umfassen Polyester, Polyamide, PVC-Copolymerisate, aliphatische und aromatische Ketonharze, Melamin-Harnstoff Harze, Melamin-Formaldehyd-Harze, Maleinate, Kolophoniumderivate, Polyvinylbutyrale, Casein bzw. Casein-Derivate, Ethylcellulose, und/oder aromatische bzw. aliphatische Polyurethane. Es können auch Polymere und/oder Copolymere von Vinylacetat, Vinylalkohol, Acrylaten, Methacrylaten, Vinylpyrrolidon und/oder Vinylacetalen eingesetzt werden.

Von besonderem Vorteil können funktionelle Gruppen aufweisende hyperverzweigte Polymere, beispielsweise hyperverzweigte Polyurethane, Polyharnstoffe oder Polyesteramide eingesetzt werden, wie in der WO 02/36695 und WO 02/36697 offenbart sind. Es können selbstverständlich auch Gemische verschiedener polymerer Bindemittel eingesetzt werden. Die Menge aller Bindemittel beträgt üblicherweise 1 bis 40 Gew.-%, vorzugsweise von 5 bis 30 Gew.-%, weiter bevorzugt von 8 bis 25 Gew.-%, bezogen auf das Gesamtgewicht des Beschichtungsmittels, vorzugsweise Druckfarbe.

Besonders bevorzugte Bindemittel umfassen beispielsweise Ethylcellulose, Hydroxyethylcellulose, Acrylate, Polyvinylbutyrale sowie aliphatische und aromatische Polyurethane und Polyharnstoffe, insbesondere hyperverzweigte Polyurethane und Polyharnstoffe oder Mischungen davon.

Ethylcellulose wird bevorzugt als Bindemittel benutzt, da es besonders geeignet ist, lagerstabile erfindungsgemäße lösemittelbasierende Druckfarben auf Basis von plättchenförmigen kupferhaltigen Effektpigmenten bereitzustellen.

Auf Ethylcellulose basierende Beschichtungsmittel, insbesondere Farben, Druckfarben oder Lacke, die Addtive mit korrosionsinhibierenden und/oder antioxidativen und/oder radikalinhibierenden Eigenschafteenthalten, zeigen eine hervorragende Lagerstabilität.

Ethylcellulose bietet zudem den Vorteil, dass damit sehr brilliante Druckfarben formuliert werden können, die insbesondere für den Direkt- und Konterdruck geeignet sind.

Die erfindungsgemäße Mischung und die erfindungsgemäßen Beschichtungsmittel, vorzugsweise Druckfarben, Farben und Lacke, können weiterhin einen oder mehrere Hilfsstoffe, die verschieden von der wenigstens einen weiteren Komponente sind, umfassen.

Beispiele für Hilfsstoffe sind Füllstoffe wie Calciumcarbonat, Aluminiumoxidhydrat, Aluminiumsilikat und/oder Magnesiumsilikat. Wachse können die Abriebfestigkeit erhöhen und dienen der Erhöhung der Gleitfähigkeit. Beispiele sind insbesondere Polyethylenwachse, oxidierte Polyethylenwachse, Petroleumwachse oder Ceresinwachse. Fettsäureamide können zur Erhöhung der Oberflächenglätte eingesetzt werden. Weichmacher dienen der Erhöhung der Elastizität des getrockneten Films.

Zum Dispergieren der Effektpigmente in der erfindungsgemäßen Mischung oder dem erfindungsgemäßen Beschichtungsmittel können Dispergierhilfsmittel eingesetzt werden.

Mittels Fettsäuren kann ein Aufschwimmen der kupferhaltigem Metalleffektpigmente in der gedruckten Schicht erreicht werden, so dass die Pigmente an der oberen Grenzfläche der Druckschicht angereichert sind. Hierdurch können vorteilhaft verbesserte Metallic-Effekte erzielt werden, Weiterhin können auch Antiabsetzmittel zugesetzt werden. Derartige Zusätze verhindern die Sedimentation der kupferhaltigen Metalleffektpigmente. Beispiele umfassen Kieselsäure, CelluloseDerivate oder auch Wachse.

Bei der Formulierung eines besonders dünnflüssigen erfindungsgemäßen Beschichtungsmittels, beispielsweise einer Druckfarbe, wie zum Beispiel von Tief- oder Flexodruckfarben, können Antiabsetzmitteln hinzugefügt werden. Die Gesamtmenge aller Hilfsstoffe sollte üblicherweise 20 Gew.-%, bezogen auf das Gesamtgewicht des Beschichtungsmittels, vorzugsweise einer Druckfarbe, nicht übersteigen und liegt bevorzugt in einem Bereich von 0,1 bis 10 Gew.-%, bezogen auf das Gesamtgewicht der Formulierung.

Die Herstellung eines erfindungsgemäßen Beschichtungsmittels, beispielsweise einer Druckfarbe, kann in prinzipiell bekannter Art und Weise durch intensives Vermischen bzw. Dispergieren der Bestandteile in üblichen Apparaturen, beispielsweise Dissolvern oder Rührwerken, erfolgen. Dabei wird beim Einsatz von Dissolvem darauf geachtet, dass der Energieeintrag nicht zu hoch ist, um ein Beschädigen der erfindungsgemäß zu verwendenden kupferhaltigen Metalleffektpigmente zu vermeiden. Der Energieintrag ist natürlich so hoch, um ein vollständiges Dispergieren der Pigmente zu ermöglichen.

Falls neben den erfindungsgemäß zu verwendenden kupferhaltigen Metalleffektpigmente noch weiter Farbpigmente eingesetzt werden, kann es empfehlenswert sein, diese in einem Teil oder in der Gesamtmenge des organischen Lösemittels, Bindemittels sowie gegebenenfalls weiterer vorhandener Hilfsstoffe vorzudispergieren und die erfindungsgemäß zu verwendenden kupferhaltigen Metalleffektpigmente erst später zuzugeben.

Auf diese Art und Weise kann eine besonders gute Dispergierung etwaig weiter verwendeter Farbpigmente erreicht werden, ohne dabei die erfindungsgemäß zu verwendenden kupferhaltigen Metalleffektpigmente durch zu starke Dispergierung zu schädigen.

Anstelle der kupferhaltigen Metalleffektpigmente können auch vordispergierte kupferhaltigen Metalleffektpigmentkonzentrate zugegeben werden.

Die vorliegende Erfindung betrifft ferner einen beschichteten Gegenstand, der eine Mischung von kupferhaltigen Metalleffektpigmenten mit wenigstens einen weiteren Komponente einem der Ansprüche 1 bis 9 oder ein Beschichtungsmittel nach einem der Ansprüche 10 bis 11 aufweist.

Bei dem Gegenstand kann es sich um Karosserie eines Automobils, ein Fassadenelement, Metall, Druckerzeugnis, Papier, Pappe, Karton, Folie, Glas, Keramik, Stein, Kunststoffe, vorzugsweise Kunststoffformteile, etc. handeln. Insbesondere kann es sich bei dem Gegenstand um Etiketten, Verpackungen, etc. handeln.

Gemäß einer bevorzugten Weiterbildung der Erfindung ist der Gegenstand transparent, vorzugsweise eine transparente Folie. Gemäß einer bevorzugten Ausführungsform wird die Folie bei der Konterapplikation verwendet. Beispielsweise eignen sich diese Konterapplikationen von Folien bei Etiketten oder Verpackungen. Bei einer Konterapplikation ist die Folienfläche dem Auge eines Betrachters zugewandt, d.h. die kupferhaltigen Metalleffektpigmente sind auf der dem Betrachter abgewandten Fläche der Folienfläche angeordnet. Eine Konterapplikation bietet den großen Vorteil, dass die kupferhaltigen Metalleffektpigmente durch die Folie vor mechanischen oder chemischen Beeinträchtigungen geschützt sind und einen hervorragenden optischen Eindruck im Hinblick auf Glanz und Farbeindruck erzeugen. Beispielsweise werden bei Etiketten für Behälter wie Getränkeflaschen, Parfümflakons, etc. die kupferhaltigen Metalleffektpigmente bzw. der Druck nicht beschädigt, wenn die Behälter aneinanderstoßen. Des weiteren ermöglicht die Konterapplikation die Bereitstellung von Etiketten und Verpackungen, die bis auf den Aufdruck der kupferhaltigen Metalleffektpigmente, transparent sind

Die Erfindung betrifft ferner ein Verfahren zur Herstellung einer Mischung von kupferhaltigen Metalleffektpigmenten mit wenigstens einer weiteren Komponente nach einem der Ansprüche 1 bis 9, wobei das Verfahren folgende Schritte umfasst:

(a) Vermahlen eines kupferhaltigen Metallgrießes mit einer Korngrößenverteilung mit einem $d_{Grieß,50}$ von 1 bis 180 $\mu$m und einem $d_{Grieß,90}$ von 2 bis 430 $\mu$m und einem Kupfergehalt von 60 bis 100 Gew.-%, bezogen auf den gesamten Metallgrieß, zu plättchenförmigen Metalleffektpigmenten unter Verwendung eines Mahlwerks in Gegenwart von Schmiermitteln und Mahlkörpern und optional Lösemittel., wobei die so erhaltenen plättchenförmigen Metalleffektpigmente eine über Dickenauszählung mit Rasterelektronenmikroskopie (REM) ermittelte mittlere Dicke mit einem $h_{50}$-Wert von 10 bis 100 nm, vorzugsweise von 10 bis 50 nm, und einem $h_{90}$-Wert von 20 bis 150 nm, vorzugsweise von 20 bis 70 nm, nm aufweisen, und

(b) in Kontakt bringen der in Schritt (a) erhaltenen plättchenförmigen Metalleffektpigmente mit wenigstens einer weiteren Komponente, die ein Cellulosederivat, ausgewählt aus der Gruppe, die aus Alkylcellulose, Hydroxyalkylcellulose, Alkyl(hydroxyalkyl)cellulose und Mischungen davon besteht, und/oder wenigstens ein Additiv mit antioxidativen und/oder radikalinhibierenden Eigenschaften ist.

Als Ausgangsmaterial bei dem Mahlschritt (Vermahlen) wird kupferhaltiger Gries, beispielsweise Kupfer- oder Messinggrieß verwendet.

Im Fall von Kupfergrieß wird vorzugsweise hochreines, elektrolytisch gewonnenes Kupfer eingesetzt. Sofern erforderlich, wird der Kupfergrieß klassiert, um einen Kupfergrieß mit der notwendigen Größenverteilung mit einem $d_{Grieß,50}$ von 1 bis 180 $\mu$m, vorzugsweise von 1 bis 15 $\mu$m und einem $d_{Grieß,90}$ von 2 bis 430 $\mu$m, vorzugsweise von 2 bis 27 $\mu$m zu erhalten.

Vorzugsweise weist der verwendete kupferhaltige Grieß, beispielsweise Messinggrieß, eine Größenverteilung mit einem $d_{Grieß,50}$ von 1 bis 180 $\mu$m und einem $d_{Grieß,90}$ von 2 bis 430 $\mu$m auf. Ein solcher Grieß wird vorzugsweise bei einer Trockenmahlung verwendet.

Weiterhin wird vorzugsweise ein kupferhaltiger Grieß, beispielsweise Messinggrieß, mit einer Größenverteilung mit einem $d_{Grieß,50}$ von 1 bis 15 $\mu$m und einem $d_{Grieß,90}$ von 2 bis 27 $\mu$m verwendet. Ein solcher Grieß wird vorzugsweise bei einer Naßmahlung verwendet.

Der kupferhaltige Metallgrieß kann neben Kupfer auch Zink und/oder Aluminium sowie weitere Metalle enthalten. Beispielsweise kann Messing 0,1 bis 2 Gew.-% Aluminium, bezogen auf das Gesamtmetallgehalt, enthalten.

Im Fall von Messinggrieß wird vorzugsweise hochreines, elektrolytisch gewonnenes Kupfer und Zink eingesetzt und vorzugsweise unter Zusatz von etwas Aluminium als Reduktionsmittel legiert. Dazu werden Kupfer und Zink miteinander verschmolzen und die erzeugte Messingschmelze zu einem Messinggrieß verdüst. Der so erhaltene Messinggrieß wird vorzugsweise klassiert, beispielsweise unter Verwendung eines Zyklons, um einen Messinggrieß mit der notwendigen Größenverteilung mit einem $d_{Grieß,50}$ von 1 bis 180 $\mu$m, vorzugsweise von 1 bis15 $\mu$m, und einem $d_{Grieß,90}$ von 2 bis 430 $\mu$m, vorzugsweise von 2 bis 27 $\mu$m, zu erhalten.

Der kupferhaltige Metallgrieß mit der erforderlichen Größenverteilung, beispielsweise ein Kupfer- oder Messinggrieß, wird anschließend zu plättchenförmigen kupferhaltigen Metalleffektpigmenten, beispielsweise Kupfer- oder Messingeffektpigmenten, vermahlen.

Die Vermahlung von kupferhaltigen Metallgrieß, beispielsweise Kupfer- oder Messinggrieß, erfolgt überwiegend nach dem Hametag'schen Trockenmahlverfahren. Hierbei wird der kupferhaltige Metallgrieß, beispielsweise Kupfer- oder Messinggrieß, in Kugelmühlen in mehreren Mahlstufen bei unterschiedlichen Mahlbedingungen, wie beispielsweise Mühlengröße, -durchmesser, -drehgeschwindigkeit, Kugelgröße, Mahldauer unter Zugabe von Schmiermittel, wie beispielsweise Stearin- oder Ölsäure, zur Verhinderung einer Kaltverschweißung der kupferhaltigen Metallpartikel, beispielsweise Kupfer- bzw. Messingpartikel, und mit Mahlkörpern, wie z. B: Stahlkugeln, vermahlen.

Bei der Trockenvermahlung von kupferhaltigem Metallgrieß, beispielsweise Kupfer- oder Messinggrieß, wird der verwendete kupferhaltige Metallgrieß, beispielsweise Kupfer- oder Messinggrieß, zu plättchenförmigen kupferhaltigen Metalleffektpigmenten vermahlen. Die relativ schwer verformbaren plättchenförmigen kupferhaltigen Metalleffektpigmente weisen eine etwa dreimal so hohe Dichte wie vergleichbare Aluminiumeffektpigmente auf. Die plättchenförmigen kupferhaltigen Metalleffektpigmente werden nach dem Vermahlen und optionalen Klassieren in verschiedenen Behältnissen gesammelt und anschließenden homogenisiert bzw. gemischt.Um den späteren metalleffektpigmentierten Beschichtungen den erforderlichen Metallglanz zu verleihen, können bei einer anschließenden Nachbearbeitung zusätzliche Hilfsstoffe (wie z.B. Stearinsäure) auf die Oberfläche der Pigmentplättchen "aufpoliert" werden.

Zur Naßvermahlung von kupferhaltigem Metallgrieß, beispielsweise Kupfer- oder Messinggrieß, wird dieser in Gegenwart von Schmier- und Lösemittel vermahlen. Eine Naßvermahlung ist bevorzugt, da diese schonender als eine Trockenvermahlung ist.

Bei einer weiteren bevorzugten Ausführungsform werden die kupferhaltigen Grießpartikel in zwei Stufen vermahlen. Dabei werden in der ersten Stufe die kupferhaltigen Grießpartikel vorverformt und in der zweiten Stufe bis zum Erhalt der vollständig flächig verformten plättchenförmigen kupferhaltigen Metalleffektpigmente vermahlen.

Der Vorverformungsschritt wird dabei vorzugsweise unter Bedingungen, die einen höheren Energieeintrag auf die kupferhaltigen Metallpartikel ermöglichen, durchgeführt.

Die beiden Stufen können beispielsweise mit unterschiedlichen Mahlkörpergrößen, beispielsweise Kugelgrößen, durchgeführt werden. Zweckmäßigerweise wird man hierbei in dem Vorverformungsschritt größere Kugeln, mit denen ein höherer Energieeintrag möglich ist, auswählen.Bei dieser Verfahrensvariante arbeitet man mithin in zwei Stufen, was aufwändiger als ein einstufiges Verfahren ist.

Bevorzugt werden daher bei einer weiteren Verfahrensvariante die beiden Stufen in einer Mühle mit derselben Mahlkörperpackung durchgeführt. Dabei kann der unterschiedliche Energieeintrag beispielsweise durch verschiedene Umdrehungsgeschwindigkeiten der Mühle und/oder durch verschiedene Mahldauern eingestellt werden.

Der zur Naßmahlung eingesetzte kupferhaltige Metallgrieß wird bevorzugt in "Atomizern" durch Verdüsung einer Kupferschmelze, einer kupferhaltigen Schmelze, beispielsweise einer Messingschmelze, beispielsweise einer Schmelze aus einer Kupfer-Zink-Legierung, hergestellt. Der nach Verdüsung einer Kupfer-, Kupferhaltigen Schmelze oder Messingschmelze erhaltene Grieß wird gemäß einer bevorzugten Variante klassiert, um die gewünschte Korngrößenverteilung, die auch als Kornband bezeichnet werden kann, zu erhalten.

Der kupferhaltige Metallgrieß, beispielsweise Kupfergrieß oder Messinggrieß, kann nach dem Verdüsungsschritt durch entsprechende Klassierungsschrrtte auf die gewünschte enge Größenverteilung gebracht werden. Die Klassierung kann mit Windsichtern, Zyklonen und anderen bekannten Einrichtungen durchgeführt werden.

Die Verwendung eines derartig feinen kupferhaltigen Metallgrießes,beispielsweise Kupfer- oder Messinggrießes, mit enger Größenverteilung ist von essentieller Bedeutung für die Herstellung der erfindungsgemäß zu verwendenden, plättchenförmigen kupferhaltigen Metalleffektpigmente,

Während der Verförmungsmahlung werden die kupferhaltigen Metallpartikel, beispielsweise Kupfer- oder Messinggrießpartikel, nicht völlig gleichmäßig verformt: Dies bedeutet, dass einige Metallpartikel stärker verformt werden, während ein Teil der Grießpartikel erst sehr spät während der Vermahlung verformt wird. Ein Grund hierfür ist auch die Tatsache, dass die Verformungswahrscheinlichkeit eines Metallpartikels von seiner Größe abhängt. So besitzen bereits zu Plättchen vorverformte Metallpartikel eine höhere spezifische Fläche als noch unverformter Metallgrieß und demgemäß eine höhere Wahrscheinlichkeit, weiter verformt zu werden. Die Breite der Größenverteilung des Metallgrießes geht somit nicht nur in die Größenverteilung der daraus geformten kupferhaltigen Metallplättchen, beispielsweise Kupfer- oder Messingplättchen, ein, sondern auch in die Verteilung der Dickenverteilung. Für enge Dickenverteilungen muss daher ein Kupfer- oder Messinggrieß mit entsprechend geringer Größenvarianz verwendet werden.

Der Verdüsungsschritt kann in Luftatmosphäre oder unter Inertgasatmosphäre durchgeführt werden. Als Inertgase werden vorzugsweise Stickstoff und/oder Helium eingesetzt.

Die Reinheit des bei der Verdüsung verwendeten Kupfers oder der Kupfer-Zink-Legierung (Messing) beträgt vorzugsweise 99,0 bis über 99,9 Gew.-%. Der Grieß kann in entsprechend geringen Mengen die üblichen Legierungsbestandteile (z.B. Al, Si,. Fe, Sn, Pb) enthalten. Bevorzugt werden 0,1 - 2 Gew.-% Aluminium zulegiert.

Die Nassvermahlung des kupferhaltigen Metallgrießes, beispielsweise Kupfer- oder Messinggrießes, erfolgt in herkömmlichen Mühlen, vorzugsweise in einer Kugelmühle, Rührwerkskugelmühle, Kollermühle, Trommelkugelmühle oder Drehrohrkugelmühle, in Gegenwart von Lösemittel und Schmiermitteln als Mahlhilfsmittel sowie unter Verwendung von Mahlkörpern.

Bei der in wenigstens zwei Schritten erfolgenden Nassvermahlung des kupferhaltigen Metallgrießes, beispielsweise Kupfer oder Messinggrießes, werden Mahlkörper, vorzugsweise kugelförmige Mahlkörper mit einem mittleren Durchmesser von 0,3 bis zu 4,7 mm und bevorzugt von 0,6 bis 2 mm eingesetzt.

Die in vielfältigen Ausführungsformen, wie beispielsweise Kugeln, Ellipsoide, Zylinder, Quader etc., eingesetzten Mahlkörper bestehen bevorzugt aus Chromstahl, Stahl, Glas oder Keramik. Besonders bevorzugt bestehen die Mahlkörper aus Chromstahl. Weiterhin werden besonders bevorzugt als Mahlkörper vorzugsweise sphärische Körper, weiter bevorzugt Kugeln, verwendet.

Bevorzugt sind kugelförmige Mahlkörper mit sehr glatter Oberfläche, möglichst runder Form und einheitlicher Größe.

Die zur Nassvermahlung des kupferhaltigen Metallgrießes, beispielsweise Kupfer- oder Messinggrießes, eingesetzten Mahlkörper weisen vorzugsweise ein Einzelgewicht von 85 $\mu$g bis 425 mg auf.

Gemäß einer bevorzugten Weiterbildung der Erfindung weisen die Mahlkörper ein Einzelgewicht von 0,8 bis 180 mg auf.

Im Falle von Stahlkugeln liegt das mittlere Einzelgewicht vorzugsweise in einem Bereich von 1 bis 180 mg, vorzugsweise von 1,2 bis 150 mg, weiter bevorzugt von 2,0 bis 120 mg. Im Falle von Glaskugeln liegt das mittlere Einzelgewicht in einem Bereich von 1,0 bis 12,5 mg.

Aufgrund der äußerst schonenden Mahlweise dauert diese Vermahlung vergleichsweise lang.

Die Mahldauer beträgt vorzugsweise 10 bis 100 Stunden, bevorzugt 20 bis 60 Stunden und besonders bevorzugt 30 bis 50 Stunden.

Diese Zeiten verstehen sich als Gesamtmahldauerzeiten.

Wenn die Mahlung in zwei oder mehr verschiedenen Schritten durchgeführt wird, so sind entsprechend die Mahldauern der einzelnen Schritte zu addieren.

Diese langen Mahldauern führen zu einer Vielzahl von Pigment-Mahlkörper-Stößen. Dadurch wird das Pigment sehr gleichmäßig ausgeformt, wodurch eine sehr glatte Oberfläche und eine sehr enge Dickenverteilung ausgebildet werden.

Dies ist bei einer Mahldauer von weniger als 10 Stunden in der Regel nicht zu erreichen. Mahldauern oberhalb von 100 Stunden hingegen sind mehr und mehr unwirtschaftlich.

Die Temperaturen während des Mahlvorganges liegen im Bereich von 15°C bis 55°C. Bevorzugt sind Temperaturen in einem Bereich von 20°C bis 35°C.

Bei der Vermahlung wird kupferhaltiges Metallpulver, beispielsweise kupferhaltiger Metallgrieß, wie Kupfer- oder Messingpulver bzw. Kupfer- oder Messinggrieß, mit bestimmter Teilchengröße zusammen mit Lösemittel, z. B. Testbenzin, in eine Kugelmühle eingetragen.

Als Lösemittel können handelsübliche, organische Lösemittel, vorzugsweise Testbenzin, Solventnaphtha, Alkohole, Glykole, Ester, Ether, Ketone oder Mischungen davon verwendet werden,

Bevorzugt sollte die Vermahlung in Lösemitteln durchgeführt werden, die kompatibel zu der später geplanten Anwendung sind.

So sind beispielsweise für eine Anwendung in einer Tiefdruckfarbe Lösemittel wie Ethylacetat, n-Propylacetat oder iso-Propylacetat bevorzugt.

Der üblicherweise bei Aluminiumpigmenten praktizierte Umnetzungsschritt ist hier nicht zu empfehlen. Beim Umnetzen werden, wenn sich dies als erforderlich erweist, die Metalleffektpigmente nach der Vermahlung unter vermindertem Druck und erhöhten Temperaturen von ihrem Lösemittel weitgehend befreit und anschließend mit dem für die jeweilige Endanwendung kompatiblen (und vom Kunden gewünschten) Lösemittel wieder angepastet.

Aufgrund der sehr hohen spezifischen Oberflächen der erfindungsgemäß zu verwendenden Metalleffektpigmente kann es bei dem Umnetzungsschritt zu unerwünschten Agglomerationen der Metallpigmente kommen. Daher sollte bevorzugt in Lösemitteln vermahlen werden, die kompatibel zu der später geplanten Anwendung sind.

Die Mahlung wird vorzugsweise in einem Lösemittel bei einem Gewichtsverhältnis von Lösemittel zu Metallpartikel von vorzugsweise 1,5:1 bis 5:1 und besonders bevorzugt von 2:1 bis 4:1 durchgeführt.

Zur Vermeidung des Kaltverschweißens der Pulverpartikel wird Schmiermittel, beispielsweise Ölsäure, Stearinsäure oder auch Inhibitoren, in einer Menge zugesetzt, welche von der jeweiligen freien spezifischen Oberfläche (BET) der ausgewalzten kupferhaltigen Metalleffektpigmente, beispielsweise Kupfer- oder Messingeffektpigmente, abhängig ist. In der Regel werden 1 bis 30 Gew.-% und bevorzugt 1,5-10 Gew.-% Schmiermittel, bezogen auf das Gewicht des kupferhaltigen Metallgrieß, beispielsweise Kupfer- oder Messinggriesses, eingesetzt.

Als Schmiermittel können während der Vermahlung eine Vielzahl von Verbindungen verwendet werden.

Hierbei sind vor allem die seit langer Zeit verwendeten Fettsäuren mit Alkylresten von 10 bis 24 C-Atomen zu nennen. Vorzugsweise wird Ölsäure oder Mischungen verschiedener ungesättigter Fettsäuren oder Mischungen von ungesättigten und gesättigten Fettsäuren verwendet, die zu non-leafing-Pigmenten führen. Non-leafing-Pigmente ordnen sich in einem Anwendungsmedium, beispielsweise einem Lack oder einer Druckfarbe - im Gegensatz zu im Anwendungsmedium an die Oberfläche aufschwimmenden leafing-Pigmenten - an. Den Fettsäuren können zusätzlich beispielsweise langkettige Aminoverbindungen zugesetzt werden. Die Fettsäuren können tierischen oder auch pflanzlichen Ursprungs sein.

Das Schmiermittel sollte in nicht zu geringer Menge eingesetzt werden, da anderenfalls infolge der starken Ausformung des kupferhaltigen Metallgrießes, beispielsweise Kupfer- oder Messinggrießes, die sehr großen Oberflächen der hergestellten plättchenartigen kupferhaltigen Metalleffektpigmente, beispielsweise Kupfer- oder Messingeffektpigmente, nur ungenügend durch adsorbiertes Schmiermittel abgesättigt werden. In diesem Fall kommt es zu Kaltverschweißungen, Typische Mengen sind daher 1 bis 30 Gew.-%, bevorzugt 2 bis 15 Gew.-% Schmiermittel bezogen auf das Gewicht des eingesetzten kupferhaltigen Metallgrießes, beispielsweise Kupfer- oder Messinggrießes.

Als besonders bevorzugtes Schmiermittel wird ein Additiv verwendet, wobei das Additiv als Struktureinheiten wenigstens eine Carbonsäure mit wenigstens 4 Kohlenstoffatomen sowie wenigstens einen Polyglykolether umfaßt, wobei die Carbonsäure und der Polyglykolether kovalent miteinander verbunden sind.

Hierbei ist besonders bevorzugt, dass die Carbonsäuren oder Fettsäuren mit einem Polyglykolether zumindest teilweise verestert sind.

So kann beispielsweise das als Prozeßhiffsmittel für Kunststoffe im Handel erhältliche Fettsäurepolyglykolester "P4100" der Fa. BYK-Chemie, Wesel, Deutschland, eingesetzt werden.

Das Gewichtsverhältnis Mahlkugeln zu Metallpartikeln beträgt vorzugsweise 10 zu 1 bis 60 zu 1, besonders bevorzugt von 25 zu 1 bis 50 zu 1.

Bezogen auf die Vermahlung in einer Kugelmühle ist die kritische Drehzahl $n_{krit}$ ein wichtiger Parameter, der angibt, ab wann die Kugeln durch die Zentrifugalkräfte an die Mühlenwand gepresst werden und praktisch keine Mahlung mehr stattfindet:

$$n_{krit} = \sqrt{\frac{g}{2\pi^2} \cdot \frac{1}{D}}$$

wobei D der Trommeldurchmesser und g die Gravitationskonstante ist.

Die Umdrehungsgeschwindigkeiten der Kugelmühle betragen vorzugsweise 20 % bis 95 %, weiter vorzugsweise 50 % bis 90 % und besonders bevorzugt 55 % bis 86 % der kritischen Drehzahl $n_{krit}$.

Die Umdrehungsgeschwindigkeiten dürfen nicht zu hoch sein, um eine langsame Verformung der Metallpartikel zu begünstigen. Andererseits benötigt der kupferhaltige Metallgrieß, insbesondere Messing- oder Goldbronzegrieß - etwa im Gegensatz zu Aluminiumgriess - aufgrund der geringeren Duktilität des Messings bzw. Kupfers oder kupferhaltiges Metall einen eher höheren Energieeintrag und daher höhere Umdrehungsgeschwindigkeiten Um eine langsame Verformung zu bewirken, werden bei dem erfindungsgemäßen Verfahren bevorzugt auch leichte Mahlkugeln verwendet.

Im Unterschied zu herkömmlichen Mahlverfahren wird der Kupfergrieß, beispielsweise kupferhaltige Metallgrieß oder Messinggrieß, bei dem erfindungsgemäßen Verfahren zum überwiegenden Anteil nicht vermahlen bzw. zerkleinert, sondern äußerst schonend über einen längeren Zeitraum verformt.

Die oben angeführten Bedingungen führen zu einer sehr schonenden Vermahlung, bei der der Metallgrieß langsam ausgeformt wird und Brüche der Metallpartikel infolge eines Kugelstoßes mit hoher kinetischer Energie vermieden werden.

Das Mahlgut wird abfiltriert und der erhaltene Filterkuchen in einer weiteren Kugelmühle mit kugelförmigen Mahlkörpern, Lösungsmittel und Mahladditiv vermahlen.

Das Mahlgut wird durch Spülen mit Lösungsmittel von den Mahlkugeln getrennt und anschließend aufkonzentriert.

In einem weiteren, bevorzugten Verfahrensschritt können die erhaltenen Metalleffektpigmente einer Größenklassifikation unterzogen werden. Diese Klassifikation sollte schonend durchgeführt werden, um die dünnen Metallpigmente nicht zu zerstören. Es kann sich dabei beispielsweise um eine Nasssiebung, eine Dekantierung oder auch um eine Trennung durch Sedimentation (infolge der Schwerkraft oder durch Zentrifugieren) handeln. Bei der Nasssiebung wird i. d. R. der Grobanteil herausgesiebt. Anschließend wird die Suspension von überschüssigem Lösemittel getrennt (z.B. mit Hilfe einer Filterpresse, Zentrifuge oder Filter).

Das Inkontaktbringen der erfindungsgemäß zu verwendenden kupferhaltigen Metalleffektpigmente mit der wenigstens einen weiteren Komponente kann durch Einbringen der kupferhaltigen Metalleffektpigmente in die wenigstens eine weitere Komponente, beispielsweise eine Lösung oder Dispersion mit der wenigstens einen weiteren Komponente erfolgen. Die wenigstens eine weitere Komponente sowie optional Bindemittel kann auch durch Aufsprühen, beispielsweise in einer Wirbelschicht, auf die erfindungsgemäß zu verwendenden kupferhaltigen Metalleffektpigmente aufgebracht werden.

Die wenigstens eine weitere Komponente, die ein Cellulosederivat, ausgewählt aus der Gruppe, die aus Alkylcellulose, Hydroxyalkylcellulose, Alkyl(hydroxyalkyl)cellulose und Mischungen davon besteht, und/oder wenigstens ein Additiv mit antioxidativen und/oder radikalinhibierenden Eigenschaften ist kann auch direkt in ein Beschichtungsmittel, beispielsweise eine Farbe oder Druckfarbe, eingebracht und nachfolgend die kupferhaltigen Metalleffektpigmente zugegeben werden.

Selbstverständlich können auch die kupferhaltigen Metalleffektpigmente in einem ersten Schritt mit der wenigstens einen Komponente zuerst in Kontakt gebracht werden und diese sodann in ein Beschichtungsmittel, beispielsweise eine Farbe oder Druckfarbe, eingebracht werden.

Auch können die kupferhaltigen Metalleffektpigmente zunächst in ein Beschichtungsmittel, beispielsweise eine Farbe oder Druckfarbe, eingearbeitet werden, sofern die wenigstens eine weitere Komponente zeitnah, vorzugsweise innerhalb von 1 h, weiter vorzugsweise innerhalb von 30 Minuten, weiter bevorzugt innerhalb von 15 Minuten, ebenfalls zu dem Beschichtungsmittel hinzugefügt wird.

Die nachfolgend aufgeführten Beispiele erläutern die Erfindung ohne sie jedoch zu beschränken:

Bei sämtlichen Beispielen und Vergleichsbeispielen wurden kupferhaltige Metalleffektpigmente verwendet, die in Form einer Paste mit einem Gehalt von 50 Gew.-% Effektpigment vorlagen. Bei den kupferhaltigen Effektpigmenten handelte es sich um non-leafing Effektpigmente mit einer Zusammensetzung von 70 Gew.-% Kupfer und 30Gew.-% zink. Das Lösemittel der Paste war Methoxypropanol. Die Pigmente wiesen einen Durchmesser mit einem $d_{50}$ von 8 $\mu$m auf. Die Dickenverteilung besaß einen $h_{10}$ von 20 nm, $h_{50}$ von 25 nm, $h_{90}$ von 30 nm und einen $h_{98}$ von 32 nm.

**Erfindungsgemäßes Beispiel 1: Herstellung einer kupferhaltigen Metalleffektpigmentpaste mit dem Radikalinhibitor BHT (Butylhydroxytoluol)**

10 g BHT (Sigma-Aldrich, St. Louis, USA) wurde in 90 g Methoxypropanol mittels eines Dispergiergerätes (Silverson L4RT; Fa. Silverson Machines Inc. UK) dispergiert. Diese Lösung wurde mit 900 Teilen der 50 Gew,%-igen kupferhaltigen Effektpigmentpaste in Methoxypropanol mittels des Mischers Visco 5000 (Collomix, D- 85080 Gaimersheim) eingebracht und homogenisiert.

**Erfindungsgemäßes Beispiel 2:**

Herstellung eines Granulats bestehend aus kupferhaltigem Effektpigment, Additiv und einem Kunstharz

15,0 g Erkamar 3300 (Robert Kraemer GmbH & Co. KG, D-26180 Rastede) wurden in 50 g Isopropylalkohol gelöst und mittels eines Dispergiergerätes (Silverson L4RT; Fa. Silverson Machines Inc. UK) homogenisiert. Diese Lösung wurde in die in Beispiel 1 genannte Mischung eingetragen und mittels eines Mischgerätes homogenisiert. Die verwendete Pastenmenge enthielt 85,0 g kupferhaltiges Effektpigment. Die homogenisierte Präparation wurde mittels einer Presse mit Stempel und Lochplatte (Eigenbau Fa. Eckart) zu Granulaten/Würstchen von einer Länge von ca. 2 - 10 mm und einem Durchmesser von ca. 2 - 3 mm verarbeitet. Die Trocknung erfolgte innerhalb von 24 Stunden bei 75°C im Vakuum (100 mbar) in einem Vakuumtrockenschrank (VDL-23, Binder GmbH, D-78532 Tuttlingen).

**Herstellung von lösemittelbasierten Tiefdruckdruckfarben aus kupferhaltigem Effektpigment Polyvinylbutyral, einem Korrosionsinhibitor und/oder einem Antioxidans, bzw. Radikalinhibitor (erfindungsgemäße Beispiele 3 bis 8)**

Allgemeine Herstellung:

Für die Herstellung der Tiefdruckfarben wurden die Lösemittel, hier 30,4 Teile Ethanol und 39 Teile Ethylacetat, vorgelegt, je 0,5 oder 1,0 Teile der jeweiligen Additive hinzugegeben, gerührt, und 3,5 Teile des Bindemittels, hier ein hochviskoses Polyvinylbutyral Pioloform BS18 (Wacker, D-84489 Burghausen), unter mittleren Drehzahlen (3000-5000 U/min) gelöst. Hierzu wurde ein Labormischer der Marke Silverson L4RT (Fa. Silverson Machines Inc. UK) benutzt. Danach wurde die kupferpigmenthaltige Effektpigmentpaste zugegeben und mit geringen-mittleren Geschwindigkeiten (3000 U/min) dispergiert.

Die fertigen Tiefdruckfarben wurden für die Druckapplikation mit einem geeigneten Lösemittel, hier Methoxypropanol, auf eine Druckviskosität zwischen von 25 sec im DIN4-Auslaufbecher nach DIN 53 211 (Fa. MTV Messtechnik, Köln) eingestellt.

Die Tiefdruckfarben wurden nun für einen Stabilitätstest für 10 Wochen bei 40°C in einem handelsüblichen Trockenschrank (Serie Heraeus Function Line T20, Heraeus Holding GmbH, D-63450 Hanau) gelagert und in regelmäßigen Abständen einer optischen und Viskositätprüfung unterzogen. Die Lagerung für 10 Wochen bei 40°C entspricht ungefähr einer Lagerung von 6 Monaten bei Raumtemperatur.

Die Tiefruckfarben werden wie vorgehend beschrieben hergestellt.

**Vergl.-Beispiel 1:** Die Tiefdruckfarbe wurde, wie oben unter dem Abschnitt "allgemeine Herstellung" beschrieben, hergestellt, jedoch ohne Additiv, d.h. ohne Antioxidans und ohne Radikalinhibitor.

**Erfindungsgemäße Beispiele 3-8:**

3.) Tiefdruckfarbe enthält den Radikalinhibitor BHT (Butylhydroxytoluol; Sigma-Aldrich, St. Louis, USA) der mit 1 Gew.-% eingesetzt wird.

4.) Tiefdruckfarbe enthält das Antioxidans $\alpha$-Tocopherol (Sigma-Aldrich, St. Louis, USA), das mit 1 Gew.-% eingesetzt wird.

5.) Tiefdruckfarbe enthält 0,5 Gew.-% Irgacor L12 als Korrosionsinhibitor und 1 Gew.-% BHT als Radikalinhibitor.

6.) Tiefdruckfarbe enthält 0,5 Gew.-% Irgacor L12 als Korrosionsinhibitor und 1 Gew.-% $\alpha$-Tocopherol als Antioxidans.

7.) Tiefdruckfarbe enthält 0,5 Gew.-% Irgamet 39 als Korrosionsinhibitor und 1 Gew.-% BHTals Radikalinhibitor.

8.) Tiefdruckfarbe enthält 0,5 Gew.-% Irgamet 39 als Korrosionsinhibitor und 1 Gew.-% $\alpha$-Tocopherol als Antixidans.

In Tab. 1 werden nun die Stabilitätsergebnisse des Vergleichsbeispiels 1 und der erfindungsgemäßen Beispiele 3 - 8 beschrieben.

**Tabelle 1: Stabilitätsergebnisse der einzelnen Druckfarben bei Lagerung bei 40°C für 10 Wochen**

| Beispiele | Viskosität nach Produktion in sec | Viskosität nach Lagerung für 10 Wochen in sec |
|---|---|---|
| Vergl.Bsp. 1 | 25 | Nicht messbar, da zu viskos |
| Erf. Bsp. 3 | 25 | 102 |
| Erf, Bsp. 4 | 25 | 112 |
| Erf. Bsp. 5 | 25 | 56 |
| Erf. Bsp. 6 | 25 | 78 |
| Erf. Bsp. 7 | 25 | 67 |
| Erf. Bsp. 8 | 25 | 78 |

Wie in Tabelle 3 beschrieben zeigen alle erfindungsgemäßen Druckfarben eine deutliche Stabilitätssteigerung. Die zu Vergleichszwecken verwendete Tiefdruckfarbe ohne Additive geliert schon nach wenigen Stunden vollständig, wohingegen die Viskosität der erfindungsgemäßen Druckfarben leicht anstieg, aber nicht gelierte.

Die erste optische Abprüfung wurde durch Auftragen der Druckfarbe auf ein geeignetes Substrat mit Hilfe eines Rakelabzugsgerätes K Control Coater 632 (Testing Machines Inc., USA) und einer Labortiefdruckmaschine Printing proofer Gravur-System Modell 628 (Erichsen, D- 58675 Hemer), durchgeführt.

Die Druckfarben wurden ebenfalls auf der Druckmaschine Rotova 300 (Rotocolor, Schweiz) auf dem Foliensubstrat PET (Melinex 400; Pütz GmbH+Co. Folien KG, D-65232 Taunusstein) angedruckt und die pigmentierten Folienkonterapplikationen wurden optisch durch eine Glanzmessung bei 60° in Anlehnung an DIN 67 530 (Gerät: microTRI-gloss von Byk-Gardner, D-82538 Geretsried) charakterisiert. Kalibriert wurde mittels Dunkelkalibrierung sowie einer schwarzen Spiegelglasplatte mit Werten von 92 für 60°.

Die Farbdichte wurde mit einem Densitometer (Gerät: Densitometer, Fa. X-Rite, D-63263 Neu-Isenburg) gemessen. Kalibriert wurde mit Hilfe eines Weißstandards und des unbedruckten Substrats.

Die Definition der Farbdichte von Druckmustem ist folgendermaßen:

$$Farbdichte = - \lg Remission$$

Dabei werden die betrachteten Oberflächen in der Aufsicht vermessen.

Die auf Grundlage von Druckmaschinenandrucken (Druckmaschine: Rotova 300, Druckverfahren: Tiefdruck; Druckgeschwindigkeit 75 m/min, Viskosität 15 s DIN-4-Auslaufbecher, 60, 70, 80 und 90 Linien/cm) ermittelten optischen Eigenschaften der Tiefdruckfarben aus den erfindungsgemäßen Beispielen 3 - 8 und Vergleichsbeispiel 1 sind in nachfolgender Tabelle 2 dargestellt.

Die Druckfarbe gemäß Vergleichsbeispiel 1 wurde für den Druckversuch frisch angesetzt.

**Tabelle 2: Optische Charatkterisierung Druckfarben**

|  | Glanz bei 60° |  |  |  | Farbdichte |  |  |  |
|---|---|---|---|---|---|---|---|---|
| **Beispiele** | **60l/cm** | **70l/cm** | **801/cml** | **90l/cm** | **60l/cm** | **70l/cm** | **80l/cm** | **90l/cm** |
| Vergl. Bsp. 1 | 407 | 408 | 399 | 384 | 1,57 | 1,65 | 1,55 | 1,45 |
| Erf. Bsp. 3 | 410 | 409 | 399 | 384 | 1,56 | 1,62 | 1,52 | 1,44 |
| Erf. Bsp. 4 | 400 | 401 | 398 | 383 | 1,56 | 1,60 | 1,45 | 1,39 |
| Erf. Bsp. 5 | 390 | 391 | 386 | 370 | 1,50 | 1,50 | 1,40 | 1,30 |
| Erf. Bsp. 6 | 391 | 392 | 380 | 375 | 1,49 | 1,51 | 1,42 | 1,29 |
| Erf. Bsp. 7 | 388 | 389 | 380 | 370 | 1,48 | 1,47 | 1,38 | 1,38 |
| Erf. Bsp. 8 | 389 | 390 | 382 | 379 | 1,49 | 1,50 | 1,43 | 1,33 |

In Tab. 2 kann eindeutig gesehen werden, dass die in den erfindungsgemäßen Druckfarben enthaltenen Additive (Komponente) nur einen geringen Einfluss auf die Optik der Druckfarben haben. Die Optik ist bei den erfindungsgemäßen Druckfarben nur leicht verschlechtert verglichen mit der Druckfarbe gemäß Vergl-Bsp.1. Der gewünschte Spiegeleffekt ist bei den erfindungsgemäßen Druckfarben nach wie vor vorhanden.

Wenn die hier beschriebenen Druckfarben einem Stabilitätstest unterzogen werden, so kann die Druckfarbe gemäß Vergl.Bsp. 1 aufgrund der Gelierung nicht mehr verdruckt werden. Die Druckfarben gemäß den erfindungsgemäßen Beispielen 1 bis 8 können ungeachtet der leichten Viskositätssteigerung unter Erzielung eines guten optischen Effektes verdruckt werden,

**Herstellung einer lösemittelbasierten Tiefdruckdruckfarbe mit kupferhaltigen Effektpigmenten, Ethylcellulose (erfindungsgemäßes Beispiel 9 und 10) oder Nitrocellulose (Vergleichsbeispiel 2 und 3) und dem Korrosionsinhibitor Irgacor L12.**

a) Druckfarbe mit dem Bindemittel Ethylcellulose (erfindungsgemäßes Beispiel 9):

Es wurde eine lösemittelbasierte Tiefdruckfarbe nach der folgenden Rezeptur hergestellt. Hierzu wurde ein Labormischer der Marke Silverson L4RT (Fa. Silverson Machines Inc. UK) benutzt.

Für die Herstellung der Druckfarbe wurde das Lösemittel, hier 25,4 Teile Ethanol und 30Teile Ethylacetat, vorgelegt, 0,6 Teile des Korrosionsinhibitor Irgacor L12 hinzugegeben, gerührt, und 4 Teile des Bindemittels, hier die hochviskose Ethylcellulose Ethocel Std 200 (Dow, Midland, USA), unter mittleren Drehzahlen (3000-5000 U/min) gelöst. Als letztes wurde die Paste (50 Gew.-% Pigment in Methoxypropanol mit d50= 8 $\mu$m und h50= 25 nm) zugegeben und mit geringen-mittleren Geschwindigkeiten (3000 U/min) dispergiert.

Die fertige Druckfarbe wurden für die Druckapplikation mit einem geeigneten Lösemittel, hier Methoxypropanol, auf eine Druckviskosität zwischen von 25 sec im DIN4-Auslaufbecher nach DIN 53 211 (Fa. MTV Messtechnik, Köln) eingestellt.

Die Druckfarbe wurde nun für einen Stabilitätstest für 10 Wochen bei 40°C in einem handelsüblichen Trockenschrank (siehe erfindungsgemäßes Beispiel 3) gelagert und in regelmäßigen Abständen einer optischen und Viskositätsprüfung unterzogen. Die Lagerung für 10 Wochen bei 40°C entspricht ungefähr einer Lagerung von 6 Monaten bei Raumtemperatur.

b) Druckfarbe mit dem Bindemittel Nitrocellulose (Vergleichsbeispiel 2)

Es wurde eine lösemittelbasierte Tiefdruckfarbe nach der folgenden Rezeptur hergestellt. Hierzu wurde ein Labormischer der Marke Silverson L4RT (Fa. Silverson Machines Inc. UK) verwendet.

Für die Herstellung der Druckfarbe wurden das Lösemittel, hier 30,4 Teile Ethanol und 39 Teile Ethylacetat, vorgelegt, 0,6 Teile des Korrosionsinhibitor Irgacor L12 und 1,0 Teile des Weichmachers Acetyltributylcitrat (Jungbunzlauer Ladenburg GmbH, D-68526 Ladenburg) hinzugegeben, gerührt, und 2,3 Teile des Bindemittels, hier die hochviskose Nitrocellulose E 1160 (Anfeuchtungsmittel: Isopropanol; Dow Wolff Cellulosics GmbH D-29656 Walsrode), unter mittleren Drehzahlen (3000-5000 U/min) gelöst. Als letztes wurde die Paste (50 Gew.-% Pigment in Methoxypropanol mit d50= 8 μm und h50= 25 nm) zugegeben und mit geringen-mittleren Geschwindigkeiten (3000 U/min) dispergiert.

Die fertige Druckfarbe wurde für die Druckapplikation mit einem geeigneten Lösemittel, hier Methoxypropanol, auf eine Druckviskosität zwischen von 25 sec im DIN4-Auslaufbecher nach DIN 53 211 (Fa. MTV Messtechnik, Köln) eingestellt.

Die Druckfarbe wurde nun für einen Stabilitätstest für 10 Wochen bei 40°C in einem handelsüblichen Trockenschrank gelagert und in regelmäßigen Abständen einer optischen und Viskositätsprüfung unterzogen. Die Lagerung für 10 Wochen bei 40°C entspricht ungefähr einer Lagerung von 6 Monaten bei Raumtemperatur.

**Erfindungsgemäßes Beispiel 9:** Druckfarbe (wie unter a) beschrieben) mit Ethylcellulose ohne Additive.

**Vergl.-Beispiel 2:** Druckfarbe mit Nitrocellulose ohne Additive (wie unter b) beschrieben).

**Erfindungsgemäßes Beispiel 10:** Druckfarbe aus Vergl.-Bsp. 1 besteht zusätzlich aus dem Korrosionsinhibitor Irgacor 12 und dem Radikalinhibitor BHT, der mit 1,0 Gew.-% eingesetzt wird (Ciba, D- 68619 Lampertheim), der mit 0,6 Gew.-% eingesetzt wird.

**Vergl.-Beispiel 3:** Druckfarbe aus Vergl.-Bsp. 2, die zusätzlich den Korrosionsinhibitor Irgacor 12 (Ciba, D-68619 Lampertheim)in einer Menge von 0,6 Gew.-% enthält.

In Tab. 3 werden nun die Stabilitätsergebnisse der beschriebenen Beispiele 9 und 10 sowie Vergleichsbeispiele 2 und 3 erläutert.

**Tabelle 3: Stabilitätsergebnisse der einzelnen Druckfarben bei Lagerung bei 40°C für 10 Wochen**

| Beispiel | Viskosität nach Produktion in sec | Viskosität nach Lagerung für 10 Wochen in sec |
|---|---|---|
| Erf. Bsp. 9 | 25 | 150 |
| Vergl.-Bsp. 2 | 25 | Nicht messbar, da zu viskos |
| Erf. Bsp. 10 | 25 | 60 |
| Vergl.-Bsp. 3 | 25 | Nicht messbar, da zu viskos |

Wie in Tabelle 3 ersichtlich, zeigen alle erfindungsgmäßen Druckfarben auf Basis des Bindemittels Ethylcellulose eine höhere Stabilität als die Druckfarben auf Basis des Bindemittels Nitrocellulose gemäß den Vergleichsbeispielen. Die Druckfarbe gemäß Vergl.-Bsp. 2 geliert schon nach einigen Stunden vollständig, wohingegen bei den erfindungsgemäßen Druckfarben auf Ethylcellulosebasis zwar die Viskosität angestiegen ist, jedoch nicht geliert sind.

Die erste optische Abprüfung wurde durch Auftragen der Druckfarbe auf ein geeignetes Substrat mit Hilfe eines Rakelabzugsgerätes K Control Coater 632 (Testing Machines Inc., USA) und einer Labortiefdruckmaschine Printing proofer Gravur-System Modell 628 (Erichsen, D-58675 Hemer), durchgeführt.

Die Druckfarben wurden auch auf der Druckmaschine Rotova 300 (Rotocolor, Schweiz) angedruckt, und die pigmentierten Folienkonterapplikationen wurden optisch durch eine Glanzmessung bei 60° in Anlehnung an DIN 67 530 (Gerät: micro-TRI-gloss von Byk-Gardner, D-82538 Geretsried) charakterisiert. Kalibriert wurde mittels Dunkelkalibrierung sowie einer schwarzen Spiegelglasplatte mit Werten von 92 für 60°.

Die Farbdichte wurde mit einem Densitometer (Gerät: Densitometer, Fa. X-Rite , D-63263 Neu-Isenburg) gemessen. Kalibriert wurde mit Hilfe eines Weißstandards und des unbedruckten Substrats.

Die Definition der Farbdichte von Druckmustern ist folgendermaßen:

$$Farbdichte = - \lg Remission$$

Dabei werden die betrachteten Oberflächen in der Aufsicht vermessen.

Die auf Grundlage von Druckmaschinenandrucken (Druckmaschine: Rotova 300, Druckverfahren: Tiefdruck; Druckgeschwindigkeit 75 m/min, Viskosität 15 s DIN-4-Auslaufbecher, 60, 70, 80 und 90 Linien/cm) ermittelten optischen Eigenschaften sind in nachfolgender Tabelle 4 dargestellt.

**Tabelle 4:Optische Charakterisierung der Druckfarben**

| | Glanz bei 60° | | | | Farbdichte | | | |
|---|---|---|---|---|---|---|---|---|
| | 60l/cm | 70l/cm | 80l/cm | 90l/cm | 60l/cm | 70l/cm | 80l/cm | 90l/cm |
| Erf. Bsp. 9 | 466 | 464 | 444 | 430 | 1,46 | 1,45 | 1,43 | 1,38 |
| Vergl.-Bsp. 2 | 458 | 458 | 434 | 424 | 1,45 | 1,46 | 1,40 | 1,34 |
| Erf. Bsp. 10 | 442 | 440 | 410 | 400 | 1,44 | 1,42 | 1,36 | 1,28 |
| Vergl.-Bsp. 3 | 400 | 399 | 398 | 390 | 1,44 | 1,46 | 1,42 | 1,30 |

In Tab. 4 kann eindeutig gesehen werden, dass die Optik der erfindungsgemäßen Druckfarbe gemäß dem erfindungsgemäßen Beispiel 10 nur einen geringfügig reduzierten Glanz aufweist:

Wenn diese Druckfarben nun für einen Stabilitätstest, wie beschrieben, gelagert werden, so können die Druckfarben gemäß den Vergleichsbeispielen 2 und 3 aufgrund der Gelierung nicht mehr verdruckt werden. Die Druckfarben gemäß den erfindungsgemäßen Beispiele 9 und 10 können trotz der Viskositätssteigerung unter Erzielung eines guten optischen Effektes verdruckt werden.

**Patentansprüche**

1.  Mischung von durch Vermahlung erhaltenen plättchenförmigen kupferhaltigen Metalleffektpigmenten, die einen Kupfergehalt von 60 bis 100 Gew.-%, bezogen auf den gesamten Metallgehalt, aufweisen, mit wenigstens einer weiteren Komponente, **dadurch gekennzeichnet, dass** die kupferhaltigen Metalleffektpigmente eine mittlere Größe $d_{50}$ von 3-50 μm und eine über Dickenauszählung mit Rasterelektronenmikroskopie (REM) ermittelte Dickenverteilung in der Darstellung als Summendurchgangsverteilung

    a) mit einem $h_{50}$-Wert von 10 bis 100 nm,
    b) mit einem $h_{50}$-Wert von 20 bis 150 nm aufweisen und dass die wenigstens eine weitere Komponente ein Cellulosederivat, ausgewählt aus der Gruppe, die aus Alkylcellulose, Hydroxyalkylcellulose, Alkyl(hydroxyalkyl)cellulose und Mischungen davon besteht, und/oder wenigstens ein Additiv mit antioxidativen und/oder radikalinhibierenden Eigenschaften ist.

2.  Mischung nach Anspruch 1,
    **dadurch gekennzeichnet**
    **dass** die kupferhaltigen Metalleffektpigmente eine über Dickenauszählung mit Rasterelektronenmikroskopie (REM) ermittelte Dickenverteilung in der Darstellung als Summendurchgangsverteilung

    a) mit einem $h_{50}$-Wert von 10 bis 50 nm,
    b) mit einem $h_{90}$-Wert von 20 bis 70 nm
    aufweisen.

3.  Mischung gemäß Anspruch 1 oder 2,
    **dadurch gekennzeichnet,**
    **dass** das Additiv ein Antioxidans und/oder Radikalfänger ist.

4.  Mischung nach einem der vorhergehenden Ansprüche,
    **dadurch gekennzeichnet,**
    **dass** in der Mischung neben der wenigstens einen weiteren Komponente ein Korrosionsinhibitor vorliegt.

5.  Mischung nach Anspruch 4,
    **dadurch gekennzeichnet,**
    **dass** der Korrosionsinhibitor aus der Gruppe, die aus Fettsäuren, Carbonsäurederivaten, organische Phosphaten und Phosphonaten und deren Estern, organisch funktionalisierten Silanen, aliphatischen oder cyclischen Aminen, aliphatischen und aromatischen Nitroverbindungen, Sauerstoff-, Schwefel- oder Stickstoff-enthaltenden Heterocyclen, Schwefel-/Stickstoffverbindungen höherer Ketone, Aldehyde und Alkohole, Thiole, ß-Diketone, ß-Ketoester und deren Gemischen besteht, ausgewählt wird.

6. Mischung nach einem der vorhergehenden Ansprüche,
**dadurch gekennzeichnet,**
**dass** das Additiv mit antioxidierenden und/oder radikalinhibierenden Eigenschaften aus der Gruppe, die aus Phenolen, Phenolderivaten, Chinonen, Chinonderivaten, Nitrosoverbindungen, Nitronen, Vitamin C, Vitamin C-Derivaten, Vitamin E, Vitamin E-Derivaten und Mischungen davon besteht, ausgewählt wird.

7. Mischung nach Anspruch 5,
**dadurch gekennzeichnet,**
**dass** das Carbonsäurederivat ein Teilester einer Dicarbonsäure, Tricarbonsäure, Tetracarbonsäure oder einer Mischung derselben ist.

8. Mischung nach einem der vorhergehenden Ansprüche,
**dadurch gekennzeichnet,**
**dass** die Mischung ein organisches Lösemittel oder organisches Lösemittelgemisch umfasst.

9. Mischung nach einem der vorhergehenden Ansprüche,
**dadurch gekennzeichnet,**
**dass** die Mischung in kompaktierter Form, vorzugsweise als Pellet, Granulat, Tabletten, Briketts und/oder Würstchen, vorliegt.

10. Beschichtungsmittel,
**dadurch gekennzeichnet,**
**dass** das Beschichtungsmittel eine Mischung nach einem vorhergehenden Ansprüche enthält und vorzugsweise eine Druckfarbe, ein Druckfarbenkonzentrat, ein Lack, ein Lackkonzentrat, eine Farbe oder ein Farbenkonzentrat ist.

11. Beschichtungsmittel nach Anspruch 10,
**dadurch gekennzeichnet,**
**dass** das Beschichtungsmittel in kompaktierter Form, vorzugsweise als Pellet, Granulat, Tabletten, Briketts und/oder Würstchen, vorliegt.

12. Beschichteter Gegenstand,
**dadurch gekennzeichnet,**
**dass** der beschichtete Gegenstand eine Mischung von kupferhaltigen Metalleffektpigmenten mit wenigstens einer Komponente nach einem der Ansprüche 1 bis 9 oder ein Beschichtungsmittel nach einem der Ansprüche 10 oder 11 aufweist.

13. Beschichteter Gegenstand nach Anspruch 12,
**dadurch gekennzeichnet,**
das der Gegenstand transparent, vorzugsweise eine transparente Folie, ist.

14. Verfahren zur Herstellung einer Mischung von kupferhaltigen Metalleffektpigmenten mit wenigstens einer Komponente nach einem der Ansprüche 1 bis 9,
**dadurch gekennzeichnet,**
**dass** das Verfahren folgende Schritte umfasst:

(a) Vermahlen eines kupferhaltigen Metallgrießes mit einer Korngrößenverteilung mit einem $d_{Grieß,50}$ von 1 bis 180 $\mu$m und einem $d_{Grieß,90}$ von 2 bis 430 $\mu$m und einem Kupfergehalt von 60 bis 100 Gew.-%, bezogen auf den gesamten Metallgrieß, zu plättchenförmigen Metalleffektpigmenten unter Verwendung eines Mahlwerks in Gegenwart von Schmiermitteln und Mahlkörpern und optional Lösemittel, wobei die so erhaltenen plättchenförmigen Metalleffektpigmente eine über Dickenauszählung mit Rasterelektronenmikroskopie (REM) ermittelte mittlere Dicke mit einem $h_{50}$-Wert von 10 bis 100 nm und einem $h_{90}$-Wert von 20 bis 150 nm aufweisen, und
(b) in Kontakt bringen der in Schritt (a) erhaltenen plättchenförmigen Metalleffektpigmente mit wenigstens einer Komponente, die ein Cellulosederivat, ausgewählt aus der Gruppe, die aus Alkylcellulose, Hydroxyalkylcellulose, Alkyl(hydroxyalkyl)cellulose und Mischungen davon besteht, und/oder wenigstens ein Additiv mit antioxidativen und/oder radikalinhibierenden Eigenschaften ist.

15. Verwendung einer Mischung von plättchenförmigen kupferhaltigen Metalleffektpigmenten nach einem der Ansprüche 1 bis 9 in einem, vorzugsweise in kompaktierter Form vorliegenden, Beschichtungsmittel.

**Claims**

1. Mixture of platelet-shaped, copper containing metal effect pigments obtained by a milling process, having a copper content of 60 to 100 % by weight based on the total metal content, having at least one other component, **characterised in that** the copper-containing metal effect pigments have a mean size $d_{50}$ of 3-50 $\mu$m and a thickness distribution determined by means of thickness counting by scanning electron microscopy (SEM) expressed as cumulative undersize distribution

   a) with an $h_{50}$ value of 10 to 100 nm,
   b) with an $h_{90}$ value of 20 to 150 nm, and the at least one other component is a cellulose derivative selected from the group comprising alkyl cellulose, hydroxyalkyl cellulose, alkyl(hydroxyalkyl) cellulose and mixtures thereof and/or at least one additive with antioxidative and/or radical-inhibiting properties.

2. Mixture as claimed in claim 1, **characterised in that** the copper-containing metal effect pigments have a thickness distribution determined by means of thickness counting by scanning electron microscopy (SEM) expressed as cumulative undersize distribution

   a) with an $h_{50}$ value of 10 to 50 nm,
   b) with an $h_{90}$ value of 20 to 70 nm.

3. Mixture as claimed in claim 1 or 2, **characterised in that** the additive is an antioxidant and/or radical scavenger.

4. Mixture as claimed in one of the preceding claims, **characterised in that** in addition to the at least one other component, there is a corrosion inhibitor in the mixture.

5. Mixture as claimed in claim 4, **characterised in that** the corrosion inhibitor is selected from the group comprising fatty acids, carboxylic acid derivatives, organic phosphates and phosphonates and esters thereof, organically functionalised silanes, aliphatic or cyclic amines, aliphatic and aromatic nitro compounds, heterocycles containing oxygen, sulphur or nitrogen, sulphur/nitrogen compounds of higher ketones, aldehydes and alcohols, thiols, $\beta$ diketones, $\beta$ keto esters and mixtures thereof.

6. Mixture as claimed in one of the preceding claims, **characterised in that** the additive with antioxidising and/or radical-inhibiting properties is selected from the group comprising phenols, phenol derivatives, quinones, quinone derivatives, nitroso compounds, nitrones, vitamin C, vitamin C derivatives, vitamin E, vitamin E derivatives and mixtures thereof.

7. Mixture as claimed in claim 5, **characterised in that** the carboxylic acid derivative is a partial ester of a dicarboxylic acid, tricarboxylic acid, tetracarboxylic acid or a mixture thereof.

8. Mixture as claimed in one of the preceding claims, **characterised in that** the mixture comprises an organic solvent or organic solvent mixture.

9. Mixture as claimed in one of the preceding claims, **characterised in that** the mixture is in compacted form, preferably in the form of pellets, granules, tablets, briquettes and/or sausages.

10. Coating composition, **characterised in that**

the coating composition contains a mixture as claimed in one of the preceding claims and is preferably a printing ink, a printing ink concentrate, a varnish, a varnish concentrate, a paint or a paint concentrate.

11. Coating composition as claimed in claim 10,
    **characterised in that**
    the coating composition is in compacted form, preferably in the form of pellets, granules, tablets, briquettes and/or sausages.

12. Coated article,
    **characterised in that**
    the coated article comprises a mixture of copper-containing metal effect pigments having at least one other component as claimed in one of claims 1 to 9 or a coating composition as claimed in one of claims 10 or 11.

13. Coated article as claimed in claim 12,
    **characterised in that**
    the article is transparent, preferably a transparent film.

14. Method of producing a mixture of copper-containing metal effect pigments having at least one other component as claimed in one of claims 1 to 9,
    **characterised in that** the method comprises the following steps:

    (a) milling a copper-containing metal powder having a particle size distribution with a $d_{powder.50}$ of 1 to 180 $\mu$m and a $d_{powder.90}$ of 2 to 430 $\mu$m and a copper content of 60 to 100 % by weight based on the total metal powder, to obtain platelet-shaped metal effect pigments using a mill in the presence of lubricants and grinding media and optionally solvent, and the platelet-shaped metal effect pigments thus obtained have a mean thickness determined by means of thickness counting by scanning electron microscopy (SEM) with an $h_{50}$ value of 10 to 100 nm and an $h_{90}$ value of 20 to 150 nm, and
    (b) placing the platelet-shaped metal effect pigments obtained in step (a) in contact with at least one component comprising a cellulose derivative selected from the group comprising alkyl cellulose, hydroxyalkyl cellulose, alkyl(hydroxyalkyl) cellulose and mixtures thereof and/or at least one additive with antioxidative and/or radical-inhibiting properties.

15. Use of a mixture of platelet-shaped copper-containing metal effect pigments as claimed in one of claims 1 to 9 in a coating composition which is preferably in compacted form.

**Revendications**

1. Mélange de pigments à effet métallique contenant du cuivre de forme plaquettaire obtenus par broyage, qui présentent une teneur en cuivre de 60 à 100 % en poids, par rapport à la teneur totale en métaux, avec au moins un autre composant, **caractérisé en ce que**
   les pigments à effet métallique contenant du cuivre présentent une taille moyenne $d_{50}$ de 3 à 50 $\mu$m et une distribution d'épaisseur calculée par dénombrement des épaisseurs par microscopie électronique à balayage (MEB) selon la représentation en tant que distribution continue cumulée présentant

    a) une valeur $h_{50}$ de 10 à 100 nm,
    b) une valeur $h_{90}$ de 20 à 150 nm, et **en ce que** ledit au moins un composant supplémentaire est un dérivé de cellulose, choisi dans le groupe constitué par l'alkylcellulose, l'hydroxyalkylcellulose, l'alkyl(hydroxyalkyl)cellulose et leurs mélanges, et/ou au moins un additif à propriétés antioxydantes et/ou d'inhibition des radicaux.

2. Mélange selon la revendication 1, **caractérisé en ce que** les pigments à effet métallique contenant du cuivre présentent une distribution d'épaisseur calculée par dénombrement des épaisseurs par microscopie électronique à balayage (MEB) selon la représentation en tant que distribution continue cumulée présentant

    a) une valeur $h_{50}$ de 10 à 50 nm,
    b) une valeur $h_{90}$ de 20 à 70 nm.

3. Mélange selon la revendication 1 ou 2, **caractérisé en ce que** l'additif est un antioxydant et/ou un capteur de radicaux.

**4.** Mélange selon l'une quelconque des revendications précédentes, **caractérisé en ce qu'**un inhibiteur de corrosion est présent dans le mélange en plus dudit au moins un composant supplémentaire.

**5.** Mélange selon la revendication 4, **caractérisé en ce que** l'inhibiteur de corrosion est choisi dans le groupe constitué par les acides gras, les dérivés d'acides carboxyliques, les phosphates organiques et les phosphonates et leurs esters, les silanes à fonctionnalisation organique, les amines aliphatiques ou cycliques, les composés nitro aliphatiques et aromatiques, les hétérocycles contenant de l'oxygène, du soufre ou de l'azote, les composés de soufre/d'azote de cétones, d'aldéhydes et d'alcools supérieurs, les thiols, les β-dicétones, les β-cétoesters et leurs mélanges.

**6.** Mélange selon l'une quelconque des revendications précédentes, **caractérisé en ce que** l'additif à propriétés antioxydantes et/ou d'inhibition des radicaux est choisi dans le groupe constitué par les phénols, les dérivés de phénols, les quinones, les dérivés de quinones, les composés nitroso, les nitrones, la vitamine C, les dérivés de vitamine C, la vitamine E, les dérivés de vitamine E et leurs mélanges.

**7.** Mélange selon la revendication 5, **caractérisé en ce que** le dérivé d'acide carboxylique est un ester partiel d'un acide dicarboxylique, d'un acide tricarboxylique, d'un acide tétracarboxylique ou d'un mélange de ceux-ci.

**8.** Mélange selon l'une quelconque des revendications précédentes, **caractérisé en ce que** le mélange comprend un solvant organique ou un mélange de solvants organique.

**9.** Mélange selon l'une quelconque des revendications précédentes, **caractérisé en ce que** le mélange se présente sous forme compactée, de préférence sous la forme de pastilles, d'un granulat, de tablettes, de briquettes et/ou de petites saucisses.

**10.** Agent de revêtement, **caractérisé en ce que** l'agent de revêtement contient un mélange selon l'une quelconque des revendications précédentes et est de préférence une encre d'impression, un concentré d'encre d'impression, un vernis, un concentré de vernis, une peinture ou un concentré de peinture.

**11.** Agent de revêtement selon la revendication 10, **caractérisé en ce que** l'agent de revêtement se présente sous forme compactée, de préférence sous la forme de pastilles, d'un granulat, de tablettes, de briquettes et/ou de petites saucisses.

**12.** Article revêtu, **caractérisé en ce que** l'article revêtu comprend un mélange de pigments à effet métallique contenant du cuivre avec au moins un composant selon l'une quelconque des revendications 1 à 9 ou un agent de revêtement selon l'une quelconque des revendications 10 ou 11.

**13.** Article revêtu selon la revendication 12, **caractérisé en ce que** l'article est transparent, de préférence une feuille transparente.

**14.** Procédé de fabrication d'un mélange de pigments à effet métallique contenant du cuivre avec au moins un composant selon l'une quelconque des revendications 1 à 9, **caractérisé en ce que** le procédé comprend les étapes suivantes :

(a) le broyage d'une semoule métallique contenant du cuivre ayant une distribution des tailles de grains qui présente un $d_{semoule,50}$ de 1 à 180 $\mu$m et un $d_{semoule,90}$ de 2 à 430 $\mu$m, et une teneur en cuivre de 60 à 100 % en poids, par rapport à la semoule métallique totale, en pigments à effet métallique de forme plaquettaire en utilisant un outil de broyage en présence de lubrifiants et de corps de broyage et éventuellement d'un solvant, les pigments à effet métallique de forme plaquettaire ainsi obtenus présentant une épaisseur moyenne calculée par dénombrement des épaisseurs par microscopie électronique à balayage (MEB) qui présente une valeur $h_{50}$ de 10 à 100 nm et une valeur $h_{90}$ de 20 à 150 nm, et

(b) la mise en contact des pigments à effets métallique de forme plaquettaire obtenus à l'étape (a) avec au moins un composant qui est un dérivé de cellulose choisi dans le groupe constitué par l'alkylcellulose, l'hydroxyalkylcellulose, l'alkyl(hydroxyalkyl)cellulose et leurs mélanges, et/ou au moins un additif à propriétés antioxydantes et/ou d'inhibition des radicaux.

**15.** Utilisation d'un mélange de pigments à effet métallique contenant du cuivre de forme plaquettaire selon l'une quelconque des revendications 1 à 9, dans un agent de revêtement, de préférence présent sous forme compacte.

**IN DER BESCHREIBUNG AUFGEFÜHRTE DOKUMENTE**

**In der Beschreibung aufgeführte Patentdokumente**

- EP 1529084 B1
- GB 994409 A
- US 6398861 B1
- WO 2006066825 A2
- DE 10315775 A1
- EP 08009699 A
- WO 0236695 A
- WO 0236697 A